(19) Europäisches Patentamt
European Patent Office
Office européen des brevets

(11) Veröffentlichungsnummer: **0 581 133 A2**

# (12) EUROPÄISCHE PATENTANMELDUNG

(21) Anmeldenummer: **93111387.2**

(22) Anmeldetag: **15.07.93**

(51) Int. Cl.5: **C07D 417/04**, C07D 249/14, A01N 43/653, A01N 43/82

(30) Priorität: **28.07.92 DE 4224929**

(43) Veröffentlichungstag der Anmeldung: **02.02.94 Patentblatt 94/05**

(84) Benannte Vertragsstaaten: **BE CH DE ES FR GB IT LI NL**

(71) Anmelder: **BAYER AG**

**D-51368 Leverkusen(DE)**

(72) Erfinder: **Haas, Wilhelm, Dr.**
**Schürgespfad 19**
**D-50259 Pulheim(DE)**
Erfinder: **Findeisen, Kurt, Prof. Dr.**
**Dünfelder Strasse 28**
**D-51375 Leverkusen(DE)**
Erfinder: **Linker, Karl-Heinz**
**Albert-Schweitzer-Strasse 3**
**d-51377 Leverkusen(DE)**
Erfinder: **Lürssen, Klaus, Dr.**
**August-Kierspel-Strasse 145**
**D-51469 Bergisch Gladbach(DE)**
Erfinder: **Santel, Hans-Joachim, Dr.**
**Grünstrasse 9a**
**D-51371 Leverkusen(DE)**
Erfinder: **Schmidt, Robert R., Dr.**
**Im Waldwinkel 110**
**D-51467 Bergisch Gladbach(DE)**

## (54) 1-Heterocyclo-substituierte-1,2,4-(1H)-Triazolin-5-one als Herbizide.

(57) Die Erfindung betrifft neue Heterocyclyltriazolinone der allgemeinen Formel (I)

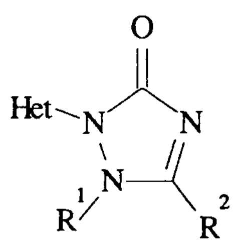

(I)

in welcher

$R^1$ für Wasserstoff oder für jeweils gegebenenfalls substituiertes Alkyl oder Cycloalkyl steht,

$R^2$ für Wasserstoff, Hydroxy, Mercapto, Halogen oder für jeweils gegebenenfalls substituiertes Alkyl, Alkenyl, Alkinyl, Cycloalkyl, Alkoxy, Alkylthio, Alkylsulfinyl, Alkylsulfonyl, Arylalkyl, Amino oder Heterocyclyl steht und

Het für einen gegebenenfalls substituierten Heterocyclus der Formel

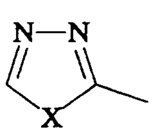 ; 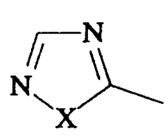 ; 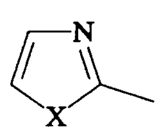 oder 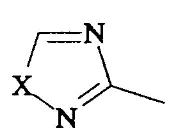

EP 0 581 133 A2

steht, wobei

X jeweils für Sauerstoff, Schwefel, eine NH-Gruppe oder eine N-Alkylgruppe steht,

ein Verfahren zu ihrer Herstellung und ihre Verwendung als Herbizide.

Die Erfindung betrifft neue Heterocyclyltriazolinone, ein Verfahren zu ihrer Herstellung und ihre Verwendung als Herbizide.

Es ist bekannt, daß Bestimmte Aminocarbonyltriazolinone wie beispielsweise die Verbindung 1-{1-[1-(2,4-Dichlorphenyl)-1-cyano]-ethyl-aminocarbonyl]}-3-dimethylamino-4-methyl-1,2,4-triazolin-5-on oder die Verbindung 1-{1-[1-(4-Chlorphenyl)-1-cyano]-propyl-aminocarbonyl]}-3-dimethylamino-4-methyl-1,2,4-triazolin-5-on herbizide Eigenschaften besitzen (vergl. z.B. EP 283 876).

Die herbizide Wirksamkeit dieser vorbekannten Verbindungen gegenüber Problemunkräutern ist jedoch ebenso wie ihre Veträglichkeit gegenüber wichtigen Kulturpflanzen nicht in allen Anwendungsgebieten völlig zufriedenstellend.

Es wurden nun neue Heterocyclyltriazolinone der allgemeinen Formel (I),

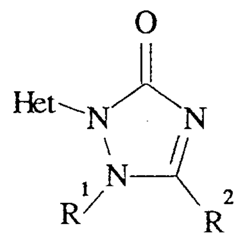

(I)

in welcher

$R^1$ für Wasserstoff oder für jeweils gegebenenfalls substituiertes Alkyl oder Cycloalkyl steht,

$R^2$ für Wasserstoff, Hydroxy, Mercapto, Halogen oder für jeweils gegebenenfalls substituiertes Alkyl, Alkenyl, Alkinyl, Cycloalkyl, Alkoxy, Alkylthio, Alkylsulfinyl, Alkylsulfonyl, Arylalkyl, Amino oder Heterocyclyl steht und

Het für einen gegebenenfalls substituierten Heterocyclus der Formel

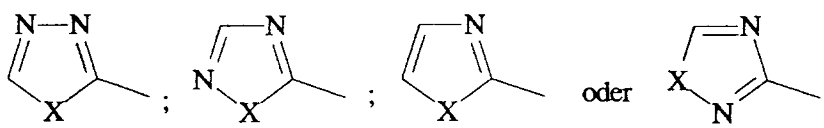

steht, wobei

X jeweils für Sauerstoff, Schwefel, eine NH-Gruppe oder eine N-Alkylgruppe steht,

gefunden.

Die Verbindungen der Formel (I) können gegebenenfalls in Abhängigkeit von der Art der Substituenten als geometrische und/oder optische Isomere oder Isomerengemische unterschiedlicher Zusammensetzung vorliegen. Sowohl die reinen Isomeren als auch die Isomerengemische werden erfindungsgemäß beansprucht.

Weiterhin wurde gefunden, daß man die neuen Heterocyclyltriazolinone der allgemeinen Formel (I),

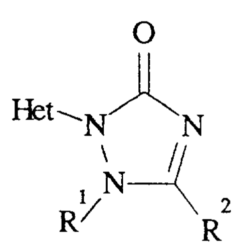

(I)

in welcher

$R^1$ für Wasserstoff oder für jeweils gegebenenfalls substituiertes Alkyl oder Cycloalkyl steht,

$R^2$ für Wasserstoff, Hydroxy, Mercapto, Halogen oder für jeweils gegebenenfalls substituiertes Alkyl, Alkenyl, Alkinyl, Cycloalkyl, Alkoxy, Alkylthio, Alkylsulfinyl, Alkylsulfonyl, Amino oder Heterocyclyl steht und

Het für einen gegebenenfalls substituierten Heterocyclus der Formel

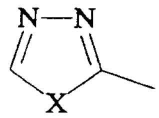 ; 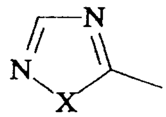 ; 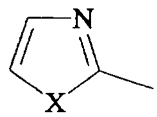 oder 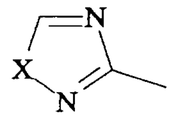

steht, wobei

X jeweils für Sauerstoff, Schwefel, eine NH-Gruppe oder eine N-Alkylgruppe steht,

erhält, wenn man Heterocyclen der Formel (II),

Het-E (II)

in welcher

Het die oben angegebene Bedeutung hat und

E für eine elektronenanziehende Abgangsgruppe steht,

mit Triazolinonen der Formel (III),

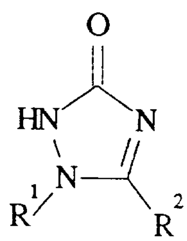 (III)

in welcher

$R^1$ und $R^2$ die oben angegebene Bedeutung haben,

gegebenenfalls in Gegenwart eines Verdünnungsmittels und gegebenenfalls in Gegenwart eines Reaktionshilfsmittels umsetzt.

Schließlich wurde gefunden, daß die neuen Heterocyclyltriazolinone der allgemeinen Formel (I) herbizide Eigenschaften besitzen.

Überraschenderweise zeigen die erfindungsgemäßen Heterocyclyltriazolinone der allgemeinen Formel (I) eine erheblich bessere herbizide Wirksamkeit gegenüber Problemunkräutern und gleichzeitig eine vergleichbar gute Verträglichkeit gegenüber wichtigen Kulturpflanzen im Vergleich zu den aus dem Stand der Technik bekannten Aminocarbonyltriazolinonen, wie beispielsweise die Verbindung 1-{1-[1-(2,4-Dichlorphenyl)-1-cyano]-ethyl-aminocarbonyl]}-3-dimethylamino-4-methyl-1,2,4-triazolin-5-on oder die Verbindung 1-{1-[1-(4-Chlorphenyl)-1-cyano]-propyl-aminocarbonyl]}-3-dimethylamino-4-methyl-1,2,4-triazolin-5-on, welche chemisch und wirkungsmäßig naheliegende Verbindungen sind.

Die erfindungsgemäßen Heterocyclyltriazolinone sind durch die Formel (I) allgemein definiert. Bevorzugt sind Verbindungen der Formel (I), bei welchen

$R^1$ für Wasserstoff oder für gegebenenfalls einfach oder mehrfach, gleich oder verschieden substituiertes, geradkettiges oder verzweigtes Alkyl mit 1 bis 6 Kohlenstoffatomen steht, wobei als Alkylsubstituenten infrage kommen:
Halogen, jeweils geradkettiges oder verzweigtes Alkoxy, Alkylthio, Alkylamino oder Dialkylamino mit jeweils 1 bis 4 Kohlenstoffatomen in den einzelnen Alkylteilen;
außerdem für Cycloalkyl mit 3 bis 8 Kohlenstoffatomen steht;

$R^2$ für Wasserstoff, Hydroxy, Mercapto, Amino, Halogen oder für gegebenenfalls einfach oder mehrfach, gleich oder verschieden substituiertes, geradkettiges oder verzweigtes Alkyl mit 1 bis 6 Kohlenstoffatomen steht, wobei als Alkylsubstituenten infrage kommen:
Halogen, Cyano sowie jeweils geradkettiges oder verzweigtes Alkoxy, Alkylthio, Alkylamino oder Dialkylamino mit jeweils 1 bis 4 Kohlenstoffatomen in den einzelnen Alkylteilen,
außerdem für jeweils geradkettiges oder verzweigtes Alkenyl oder Alkinyl mit jeweils 2 bis 6 Kohlenstoffatomen, für jeweils geradkettiges oder verzweigtes Alkoxy, Alkylthio, Alkylsulfinyl oder Alkylsulfonyl mit jeweils 1 bis 6 Kohlenstoffatomen oder für Cycloalkyl mit 3 bis 8 Kohlenstoffatomen steht;
außerdem für gegebenenfalls im Arylteil einfach oder mehrfach, gleich oder verschieden

substituiertes Arylalkyl mit 6 bis 10 Kohlenstoffatomen im Arylteil und 1 bis 6 Kohlenstoffatomen im geradkettigen oder verzweigten Alkylteil steht, wobei als Arylsubstituenten infrage kommen:
Halogen, Cyano, Nitro, jeweils geradkettiges oder verzweigtes Alkyl, Alkoxy oder Alkylthio mit jeweils 1 bis 4 Kohlenstoffatomen, jeweils geradkettiges oder verzweigtes Halogenalkyl, Halogenalkoxy, Halogenalkylthio mit jeweils 1 bis 4 Kohlenstoffatomen und 1 bis 9 gleichen oder verschiedenen Halogenatomen, jeweils geradkettiges oder verzweigtes Alkoxycarbonyl oder Alkoximinoalkyl mit jeweils 1 bis 4 Kohlenstoffatomen in den einzelnen Alkylteilen sowie gegebenenfalls einfach oder mehrfach, gleich oder verschieden durch Halogen und/oder geradkettiges oder verzweigtes Alkyl mit 1 bis 4 Kohlenstoffatomen substituiertes Phenyl;
und außerdem für einen Rest

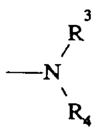

steht,

Het für einen gegebenenfalls einfach oder mehrfach, gleich oder verschieden substituierten Heterocyclus der Formel

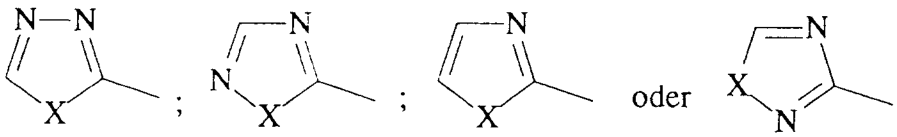

; ; oder

steht,
wobei als Substituenten jeweils infrage kommen:
Halogen, Hydroxy, Mercapto, Amino, Cyano, Nitro, Carbamoyl, jeweils geradkettiges oder verzweigtes Alkyl, Alkoxy, Alkylthio, Alkylsulfinyl, Alkylsulfonyl, Alkylamino, Dialkylamino, Alkanoylamino; Halogenalkylcarbonylamino oder Alkylaminocarbonylamino mit jeweils 1 bis 8 Kohlenstoffatomen in den einzelnen Alkylteilen und gegebenenfalls 1 bis 13 gleichen oder verschiedenen Halogenatomen, jeweils geradkettiges oder verzweigtes Alkenyl oder Alkinyl mit jeweils 2 bis 6 Kohlenstoffatomen, geradkettiges oder verzweigtes Alkylidenamino mit 1 bis 6 Kohlenstoffatomen, jeweils geradkettiges oder verzweigtes Halogenalkyl, Halogenalkoxy, Halogenalkylthio, Halogenalkylsulfinyl oder Halogenalkylsulfonyl mit jeweils 1 bis 6 Kohlenstoffatomen und 1 bis 13 gleichen oder verschiedenen Halogenatomen, geradkettiges oder verzweigtes Alkoxycarbonyl mit 1 bis 7 Kohlenstoffatomen, Cycloalkyl mit 3 bis 8 Kohlenstoffatomen sowie jeweils gegebenenfalls im Arylteil einfach oder mehrfach, gleich oder verschieden durch Halogen und/oder geradkettiges oder verzweigtes Alkyl und/oder Alkoxy mit jeweils 1 bis 4 Kohlenstoffatomen und/oder Halogenalkyl mit 1 bis 4 Kohlenstoffatomen und 1 bis 9 gleichen oder verschiedenen Halogenatomen substituiertes Aryl, Arylalkyl, Arylalkylidenamino oder Arylalkylaminocarbonylamino mit jeweils 6 bis 10 Kohlenstoffatomen im Arylteil und gegebenenfalls 1 bis 4 Kohlenstoffatomen im geradkettigen oder verzweigten Alkylteil und

X jeweils für Sauerstoff, Schwefel, eine NH-Gruppe oder eine geradkettige oder verzweigte N-Alkylgruppe mit 1 bis 8 Kohlenstoffatomen steht,

$R^3$ für Wasserstoff oder für gegebenenfalls einfach oder mehrfach, gleich oder verschieden substituiertes, geradkettiges oder verzweigtes Alkyl mit 1 bis 6 Kohlenstoffatomen steht, wobei als Alkylsubstituenten infrage kommen:
Halogen, Cyano, jeweils geradkettiges oder verzweigtes Alkoxy, Alkylthio, Alkylamino oder Dialkylamino mit jeweils 1 bis 4 Kohlenstoffatomen in den einzelnen Alkylteilen sowie gegebenenfalls einfach oder mehrfach, gleich oder verschieden durch Halogen und/oder geradkettiges oder verzweigtes Alkyl mit 1 bis 4 Kohlenstoffatomen substituiertes Phenyl;
außerdem für jeweils geradkettiges oder verzweigtes Alkenyl oder Alkinyl mit jeweils 2 bis

6 Kohlenstoffatomen, für geradkettiges oder verzweigtes Alkoxy mit 1 bis 6 Kohlenstoffatomen oder für Cycloalkyl mit 3 bis 8 Kohlenstoffatomen steht und

$R^4$ für gegebenenfalls einfach oder mehrfach, gleich oder verschieden substituiertes, geradkettiges oder verzweigtes Alkyl mit 1 bis 6 Kohlenstoffatomen steht, wobei als Alkylsubstituenten infrage kommen:
Halogen, Cyano, jeweils geradkettiges oder verzweigtes Alkoxy, Alkylthio, Alkylamino oder Dialkylamino mit jeweils 1 bis 4 Kohlenstoffatomen in den einzelnen Alkylteilen sowie gegebenenfalls einfach oder mehrfach, gleich oder verschieden durch Halogen und/oder geradkettiges oder verzweigtes Alkyl mit 1 bis 4 Kohlenstoffatomen substituiertes Phenyl;
außerdem für jeweils geradkettiges oder verzweigtes Alkenyl oder Alkinyl mit jeweils 2 bis 6 Kohlenstoffatomen, für geradkettiges oder verzweigtes Alkoxy mit 1 bis 6 Kohlenstoffatomen oder für Cycloalkyl mit 3 bis 8 Kohlenstoffatomen steht oder

$R^3$ und $R^4$ gemeinsam mit dem Stickstoffatom, an welches sie gebunden sind, für einen gegebenenfalls einfach oder mehrfach, gleich oder verschieden substituierten, gesättigten oder ungesättigten Heterocyclus stehen der gegebenenfalls 1 bis 3 weitere Heteroatome - insbesondere Stickstoff, Sauerstoff und/oder Schwefel - enthalten kann, wobei als Heterocyclylsubstituenten jeweils infrage kommen:
Halogen, Hydroxy, Mercapto, Amino, Cyano, Nitro, Carbamoyl, jeweils geradkettiges oder verzweigtes Alkyl, Alkoxy, Alkylthio, Alkylsulfinyl, Alkylsulfonyl, Alkylamino, Dialkylamino, Alkanoylamino; Halogenalkylcarbonylamino oder Alkylaminocarbonylamino mit jeweils 1 bis 8 Kohlenstoffatomen in den einzelnen Alkylteilen und gegebenenfalls 1 bis 13 gleichen oder verschiedenen Halogenatomen, jeweils geradkettiges oder verzweigtes Alkenyl oder Alkinyl mit jeweils 2 bis 6 Kohlenstoffatomen, geradkettiges oder verzweigtes Alkylidenamino mit 1 bis 6 Kohlenstoffatomen, jeweils geradkettiges oder verzweigtes Halogenalkyl, Halogenalkoxy, Halogenalkylthio, Halogenalkylsulfinyl oder Halogenalkylsulfonyl mit jeweils 1 bis 6 Kohlenstoffatomen und 1 bis 13 gleichen oder verschiedenen Halogenatomen, geradkettiges oder verzweigtes Alkoxycarbonyl mit 1 bis 7 Kohlenstoffatomen, Cycloalkyl mit 3 bis 8 Kohlenstoffatomen sowie jeweils gegebenenfalls im Arylteil einfach oder mehrfach, gleich oder verschieden durch Halogen und/oder geradkettiges oder verzweigtes Alkyl und/oder Alkoxy mit jeweils 1 bis 4 Kohlenstoffatomen und/oder Halogenalkyl mit 1 bis 4 Kohlenstoffatomen und 1 bis 9 gleichen oder verschiedenen Halogenatomen substituiertes Aryl, Arylalkyl, Arylalkylidenamino oder Arylalkylaminocarbonylamino mit jeweils 6 bis 10 Kohlenstoffatomen im Arylteil und gegebenenfalls 1 bis 4 Kohlenstoffatomen im geradkettigen oder verzweigten Alkylteil.

Besonders bevorzugt sind Verbindungen der Formel (I), bei welchen

$R^1$ für Wasserstoff oder für gegebenenfalls einfach bis zweifach, gleich oder verschieden substituiertes, geradkettiges oder verzweigtes Alkyl mit 1 bis 4 Kohlenstoffatomen steht, wobei als Alkylsubstituenten infrage kommen:
jeweils geradkettiges oder verzweigtes Alkoxy, Alkylthio, Alkylamino oder Dialkylamino mit jeweils 1 bis 3 Kohlenstoffatomen in den einzelnen Alkylteilen;
außerdem für geradkettiges oder verzweigtes Halogenalkyl mit 1 bis 4 Kohlenstoffatomen und 1 bis 9 gleichen oder verschiedenen Halogenatomen - insbesondere Fluor, Chlor und/oder Brom- steht, außerdem für Cycloalkyl mit 3 bis 7 Kohlenstoffatomen steht;

$R^2$ für Wasserstoff, Hydroxy, Mercapto, Amino, Halogen oder für gegebenenfalls einfach oder zweifach, gleich oder verschieden substituiertes, geradkettiges oder verzweigtes Alkyl mit 1 bis 4 Kohlenstoffatomen steht, wobei als Alkylsubstituenten infrage kommen:
Cyano oder jeweils geradkettiges oder verzweigtes Alkoxy, Alkylthio, Alkylamino oder Dialkylamino mit jeweils 1 bis 3 Kohlenstoffatomen in den einzelnen Alkylteilen,
außerdem für geradkettiges oder verzweigtes Halogenalkyl mit 1 bis 4 Kohlenstoffatomen und 1 bis 9 gleichen oder verschiedenen Halogenatomen - insbesondere Fluor, Chlor und/oder Brom- steht, außerdem für jeweils geradkettiges oder verzweigtes Alkenyl oder Alkinyl mit jeweils 2 bis 5 Kohlenstoffatomen, für jeweils geradkettiges oder verzweigtes Alkoxy, Alkylthio, Alkylsulfinyl oder Alkylsulfonyl mit jeweils 1 bis 4 Kohlenstoffatomen oder für Cycloalkyl mit 3 bis 7 Kohlenstoffatomen steht;
außerdem für gegebenenfalls im Arylteil einfach bis dreifach, gleich oder verschieden substituiertes Arylalkyl mit 6 oder 10 Kohlenstoffatomen im Arylteil und 1 bis 4 Kohlenstoffatomen im geradkettigen oder verzweigten Alkylteil steht, wobei als Arylsubstituenten infrage kommen:

Halogen, Cyano, Nitro, jeweils geradkettiges oder verzweigtes Alkyl, Alkoxy oder Alkylthio mit jeweils 1 bis 3 Kohlenstoffatomen, jeweils geradkettiges oder verzweigtes Halogenalkyl, Halogenalkoxy, Halogenalkylthio mit jeweils 1 bis 3 Kohlenstoffatomen und 1 bis 7 gleichen oder verschiedenen Halogenatomen, jeweils geradkettiges oder verzweigtes Alkoxycarbonyl oder Alkoximinoalkyl mit jeweils 1 bis 3 Kotlenstoffatomen in den einzelnen Alkylteilen sowie gegebenenfalls einfach bis dreifach, gleich oder verschieden durch Halogen und/oder geradkettiges oder verzweigtes Alkyl mit 1 bis 3 Kohlenstoffatomen substituiertes Phenyl;
und außerdem für einen Rest

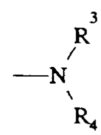

steht,

Het für einen gegebenenfalls einfach bis zweifach, gleich oder verschieden substituierten Heterocyclus der Formel

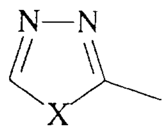 ; 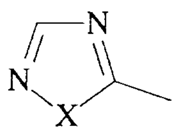 ; 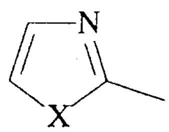 oder 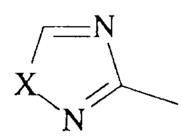

steht,
wobei als Substituenten jeweils infrage kommen:
Halogen, Hydroxy, Mercapto, Amino, Cyano, Nitro, Carbamoyl, jeweils geradkettiges oder verzweigtes Alkyl, Alkoxy, Alkylthio, Alkylsulfinyl, Alkylsulfonyl, Alkylamino, Dialkylamino, Alkanoylamino, Halogenalkylcarbonylamino oder Alkylaminocarbonylamino mit jeweils 1 bis 6 Kohlenstoffatomen in den einzelnen Alkylteilen und gegebenenfalls 1 bis 13 gleichen oder verschiedenen Halogenatomen - insbesondere Fluor, Chlor und/oder Brom -, jeweils geradkettiges oder verzweigtes Alkenyl oder Alkinyl mit jeweils 2 bis 5 Kohlenstoffatomen, jeweils geradkettiges oder verzweigtes Alkylidenamino mit 1 bis 4 Kohlenstoffatomen, jeweils geradkettiges oder verzweigtes Halogenalkyl, Halogenalkoxy, Halogenalkylthio, Halogenalkylsulfinyl oder Halogenalkylsulfonyl mit jeweils 1 bis 4 Kohlenstoffatomen und 1 bis 9 gleichen oder verschiedenen Halogenatomen, geradkettiges oder verzweigtes Alkoxycarbonyl mit 1 bis 5 Kohlenstoffatomen, Cycloalkyl mit 3 bis 7 Kohlenstoffatomen sowie jeweils gegebenenfalls im Arylteil einfach bis dreifach, gleich oder verschieden durch Halogen und/oder geradkettiges oder verzweigtes Alkyl und/oder Alkoxy mit jeweils 1 bis 3 Kohlenstoffatomen und/oder Halogenalkyl mit 1 bis 3 Kohlenstoffatomen und 1 bis 7 gleichen oder verschiedenen Halogenatomen substituiertes Aryl, Arylalkyl, Arylalkylidenamino oder Arylalkylaminocarbonylamino mit jeweils 6 oder 10 Kohlenstoffatomen im Arylteil und gegebenenfalls 1 bis 3 Kohlenstoffatomen im geradkettigen oder verzweigten Alkylteil und

X jeweils für Sauerstoff, Schwefel, eine NH-Gruppe oder eine geradkettige oder verzweigte N-Alkylgruppe mit 1 bis 6 Kohlenstoffatomen steht,

$R^3$ für Wasserstoff oder für gegebenenfalls einfach bis zweifach, gleich oder verschieden substituiertes, geradkettiges oder verzweigtes Alkyl mit 1 bis 4 Kohlenstoffatomen steht, wobei als Alkylsubstituenten infrage kommen:
Cyano, jeweils geradkettiges oder verzweigtes Alkoxy, Alkylthio, Alkylamino oder Dialkylamino mit jeweils 1 bis 3 Kohlenstoffatomen in den einzelnen Alkylteilen sowie gegebenenfalls einfach bis dreifach, gleich oder verschieden durch Halogen und/oder geradkettiges oder verzweigtes Alkyl mit 1 bis 3 Kohlenstoffatomen substituiertes Phenyl;
außerdem für geradkettiges oder verzweigtes Halogenalkyl mit 1 bis 4 Kohlenstoffatomen und 1 bis 9 gleichen oder verschiedenen Halogenatomen - insbesondere Fluor, Chlor und/oder Brom- steht, außerdem für jeweils geradkettiges oder verzweigtes Alkenyl oder

Alkinyl mit jeweils 2 bis 5 Kohlenstoffatomen, für geradkettiges oder verzweigtes Alkoxy mit 1 bis 4 Kohlenstoffatomen oder für Cycloalkyl mit 3 bis 7 Kohlenstoffatomen steht und

$R^4$ für gegebenenfalls einfach oder zweifach, gleich oder verschieden substituiertes, geradkettiges oder verzweigtes Alkyl mit 1 bis 4 Kohlenstoffatomen steht, wobei als Alkylsubstituenten infrage kommen:

Cyano, jeweils geradkettiges oder verzweigtes Alkoxy, Alkylthio, Alkylamino oder Dialkylamino mit jeweils 1 bis 3 Kohlenstoffatomen in den einzelnen Alkylteilen sowie gegebenenfalls einfach bis dreifach, gleich oder verschieden durch Halogen und/oder geradkettiges oder verzweigtes Alkyl mit 1 bis 3 Kohlenstoffatomen substituiertes Phenyl;

außerdem für geradkettiges oder verzweigtes Halogenalkyl mit 1 bis 4 Kohlenstoffatomen und 1 bis 9 gleichen oder verschiedenen Halogenatomen - insbesondere Fluor, Chlor und/oder Brom- steht, außerdem für jeweils geradkettiges oder verzweigtes Alkenyl oder Alkinyl mit jeweils 2 bis 5 Kohlenstoffatomen, für geradkettiges oder verzweigtes Alkoxy mit 1 bis 4 Kohlenstoffatomen oder für Cycloalkyl mit 3 bis 7 Kohlenstoffatomen steht oder

$R^3$ und $R^4$ gemeinsam mit dem Stickstoffatom, an welches sie gebunden sind, für einen gegebenenfalls einfach oder zweifach, gleich oder verschieden substituierten, gesättigten fünf- bis siebengliedrigen, monocyclischen Heterocyclus stehen der gegebenenfalls 1 oder 2 weitere Heteroatome - insbesondere Stickstoff, Sauerstoff und/oder Schwefel - enthalten kann, wobei als Heterocyclylsubstituenten jeweils infrage kommen:

Halogen, Hydroxy, Mercapto, Amino, Cyano, Nitro, Carbamoyl, jeweils geradkettiges oder verzweigtes Alkyl, Alkoxy, Alkylthio, Alkylsulfinyl, Alkylsulfonyl, Alkylamino, Dialkylamino, Alkanoylamino, Halogenalkylcarbonylamino oder Alkylaminocarbonylamino mit jeweils 1 bis 6 Kohlenstoffatomen in den einzelnen Alkylteilen und gegebenenfalls 1 bis 13 gleichen oder verschiedenen Halogenatomen - insbesondere Fluor, Chlor und/oder Brom -, jeweils geradkettiges oder verzweigtes Alkenyl oder Alkinyl mit jeweils 2 bis 5 Kohlenstoffatomen, geradkettiges oder verzweigtes Alkylidenamino mit 1 bis 4 Kohlenstoffatomen, jeweils geradkettiges oder verzweigtes Halogenalkyl, Halogenalkoxy, Halogenalkylthio, Halogenalkylsulfinyl oder Halogenalkylsulfonyl mit jeweils 1 bis 4 Kohlenstoffatomen und 1 bis 9 gleichen oder verschiedenen Halogenatomen, geradkettiges oder verzweigtes Alkoxycarbonyl mit 1 bis 5 Kohlenstoffatomen, Cycloalkyl mit 3 bis 7 Kohlenstoffatomen sowie jeweils gegebenenfalls im Arylteil einfach bis dreifach, gleich oder verschieden durch Halogen und/oder geradkettiges oder verzweigtes Alkyl und/oder Alkoxy mit jeweils 1 bis 3 Kohlenstoffatomen und/oder Halogenalkyl mit 1 bis 3 Kohlenstoffatomen und 1 bis 7 gleichen oder verschiedenen Halogenatomen substituiertes Aryl, Arylalkyl, Arylalkylidenamino oder Arylalkylaminocarbonylamino mit jeweils 6 oder 10 Kohlenstoffatomen im Arylteil und gegebenenfalls 1 bis 3 Kohlenstoffatomen im geradkettigen oder verzweigten Alkylteil.

Ganz besonders bevorzugt sind Verbindungen der Formel (I), bei welchen

$R^1$ für Wasserstoff oder für gegebenenfalls einfach substituiertes, geradkettiges oder verzweigtes Alkyl mit 1 bis 3 Kohlenstoffatomen steht, wobei als Alkylsubstituenten infrage kommen:

Alkoxy, Alkylthio, Alkylamino oder Dialkylamino mit jeweils 1 oder 2 Kohlenstoffatomen in den einzelnen Alkylteilen;

außerdem für geradkettiges oder verzweigtes Halogenalkyl mit 1 bis 3 Kohlenstoffatomen und 1 bis 7 gleichen oder verschiedenen Halogenatomen - insbesondere Fluor Chlor und/oder Brom- steht oder für Cycloalkyl mit 3 bis 6 Kohlenstoffatomen steht;

$R^2$ für Wasserstoff, Hydroxy, Mercapto, Amino, Fluor, Chlor, Brom oder für gegebenenfalls einfach substituiertes, geradkettiges oder verzweigtes Alkyl mit 1 bis 3 Kohlenstoffatomen steht, wobei als Alkylsubstituenten infrage kommen:

Cyano sowie Alkoxy, Alkylthio, Alkylamino oder Dialkylamino mit jeweils 1 oder 2 Kohlenstoffatomen in den einzelnen Alkylteilen;

außerdem für geradkettiges oder verzweigtes Halogenalkyl mit 1 bis 3 Kohlenstoffatomen und 1 bis 7 gleichen oder verschiedenen Halogenatomen - insbesondere Fluor, Chlor und/oder Brom- steht, außerdem für jeweils geradkettiges oder verzweigtes Alkenyl oder Alkinyl mit jeweils 2 bis 4 Kohlenstoffatomen, für jeweils geradkettiges oder verzweigtes Alkoxy, Alkylthio, Alkylsulfinyl oder Alkylsulfonyl mit jeweils 1 bis 3 Kohlenstoffatomen oder für Cycloalkyl mit 3 bis 6 Kohlenstoffatomen steht;

außerdem für gegebenenfalls im Phenylteil einfach oder zweifach, gleich oder verschieden

substituiertes Phenylalkyl mit 1 oder 2 Kohlenstoffatomen im Alkylteil steht, wobei als Phenylsubstituenten infrage kommen:
Halogen, Cyano, Nitro, Alkyl, Alkoxy oder Alkylthio mit jeweils 1 oder 2 Kohlenstoffatomen, Halogenalkyl, Halogenalkoxy oder Halogenalkylthio mit jeweils 1 oder 2 Kohlenstoffatomen und 1 bis 5 gleichen oder verschiedenen Halogenatomen - insbesondere Fluor, Chlor und/oder Brom - , Alkoxycarbonyl oder Alkoximinoalkyl mit 1 bis 3 Kohlenstoffatomen sowie gegebenenfalls einfach oder zweifach, gleich oder verschieden durch Fluor, Chlor, Brom, Methyl und/oder Ethyl substituiertes Phenyl;
und außerdem für einen Rest

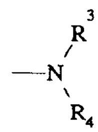

steht,

Het für einen gegebenenfalls einfach oder zweifach, gleich oder verschieden substituierten Heterocyclus der Formel

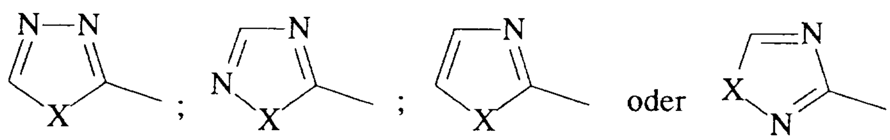

steht,
wobei als Substituenten jeweils infrage kommen:
Halogen, Hydroxy, Mercapto, Amino, Cyano, Nitro, Carbamoyl, jeweils geradkettiges oder verzweigtes Alkyl, Alkoxy, Alkylthio, Alkylsulfinyl, Alkylsulfonyl, Alkylamino, Dialkylamino, Alkanoylamino, Halogenalkylcarbonylamino oder Alkylaminocarbonylamino mit jeweils 1 bis 4 Kohlenstoffatomen in den einzelnen Alkylteilen und gegebenenfalls 1 bis 7 gleichen oder verschiedenen Halogenatomen - insbesondere Fluor, Chlor und/oder Brom -, jeweils geradkettiges oder verzweigtes Alkenyl oder Alkinyl mit jeweils 2 bis 4 Kohlenstoffatomen, geradkettiges oder verzweigtes Alkylidenamino mit 1 bis 3 Kohlenstoffatomen, jeweils geradkettiges oder verzweigtes Halogenalkyl, Halogenalkoxy, Halogenalkylthio, Halogenalkylsulfinyl oder Halogenalkylsulfonyl mit jeweils 1 bis 3 Kohlenstoffatomen und 1 bis 7 gleichen oder verschiedenen Halogenatomen, geradkettiges oder verzweigtes Alkoxycarbonyl mit 1 bis 4 Kohlenstoffatomen, Cycloalkyl mit 3 bis 6 Kohlenstoffatomen sowie jeweils gegebenenfalls im Phenylteil einfach oder zweifach, gleich oder verschieden durch Fluor, Chlor, Brom, Methyl, Ethyl, Methoxy, Ethoxy und/oder Trifluormethyl substituiertes Phenyl, Benzyl, Phenylethyl, Benzylidenamino oder Benzylaminocarbonylamino und

X für Sauerstoff, Schwefel, eine NH-Gruppe eine N-Methylgruppe oder eine N-Ethylgruppe steht, wobei

$R^3$ für Wasserstoff oder für gegebenenfalls einfach substituiertes, geradkettiges oder verzweigtes Alkyl mit 1 bis 3 Kohlenstoffatomen steht, wobei als Alkylsubstituenten infrage kommen:
Cyano, Alkoxy, Alkylthio, Alkylamino oder Dialkylamino mit jeweils 1 oder 2 Kohlenstoffatomen in den einzelnen Alkylteilen sowie gegebenenfalls einfach oder zweifach, gleich oder verschieden durch Fluor, Chlor Brom, Methyl und/oder Ethyl substituiertes Phenyl;
außerdem für geradkettiges oder verzweigtes Halogenalkyl mit 1 bis 3 Kohlenstoffatomen und 1 bis 7 gleichen oder verschiedenen Halogenatomen - insbesondere Fluor, Chlor und/oder Brom- steht, außerdem für jeweils geradkettiges oder verzweigtes Alkenyl oder Alkinyl mit jeweils 2 bis 4 Kohlenstoffatomen, für geradkettiges oder verzweigtes Alkoxy mit 1 bis 3 Kohlenstoffatomen oder für Cycloalkyl mit 3 bis 6 Kohlenstoffatomen steht und

$R^4$ für gegebenenfalls einfach substituiertes, geradkettiges oder verzweigtes Alkyl mit 1 bis 3 Kohlenstoffatomen steht, wobei als Alkylsubstituenten infrage kommen:
Cyano, Alkoxy, Alkylthio, Alkylamino oder Dialkylamino mit jeweils 1 oder 2 Kohlenstoffatomen in den einzelnen Alkylteilen sowie gegebenenfalls einfach oder zweifach, gleich oder verschieden durch Fluor, Chlor, Brom, Methyl und/oder Ethyl substituiertes Phenyl;
außerdem für geradkettiges oder verzweigtes Halogenalkyl mit 1 bis 3 Kohlenstoffatomen und 1 bis 7 gleichen oder verschiedenen Halogenatomen - insbesondere Fluor, Chlor und/oder Brom- steht, außerdem für jeweils geradkettiges oder verzweigtes Alkenyl oder Alkinyl mit jeweils 2 bis 4 Kohlenstoffatomen, für geradkettiges oder verzweigtes Alkoxy mit 1 bis 3 Kohlenstoffatomen oder für Cycloalkyl mit 3 bis 6 Kohlenstoffatomen steht oder

$R^3$ und $R^4$ gemeinsam mit dem Stickstoffatom, an welches sie gebunden sind, für einen gegebenenfalls einfach oder zweifach, gleich oder verschieden durch Methyl und/oder Ethyl und/oder Methoxy substituierten 1-Pyrrolidinyl-, 1-Piperidinyl, 1-Perhydroazepinyl oder 4-Morpholinylrest stehen.

Im einzelnen seien außer den bei den Herstellungsbeispielen genannten Verbindungen die folgenden Heterocyclyltriazolinone der allgemeinen Formel (I) genannt:

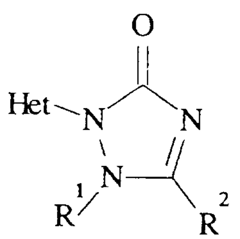

(I)

| $R^1$ | $R^2$ | Het |
|---|---|---|
| $CH_3$ | $N(CH_3)_2$ | 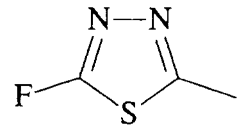 |
| $CH_3$ | $N(CH_3)_2$ | 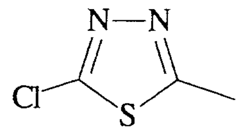 |
| $CH_3$ | $N(CH_3)_2$ | 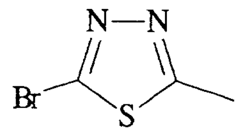 |
| $CH_3$ | $N(CH_3)_2$ | 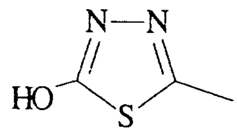 |
| $CH_3$ | $N(CH_3)_2$ | 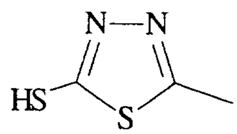 |
| $C_2H_5$ | $N(CH_3)_2$ | 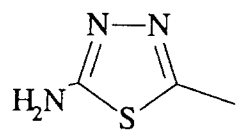 |
| $CH_3$ | $N(CH_3)_2$ | 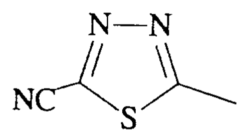 |
| $CH_3$ | $N(CH_3)_2$ | 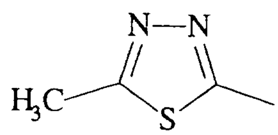 |

| $R^1$ | $R^2$ | Het |
|---|---|---|
| $CH_3$ | $N(CH_3)_2$ | 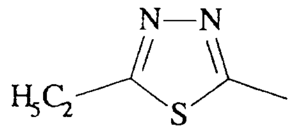 |
| $CH_3$ | $N(CH_3)_2$ | 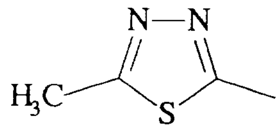 |
| $CH_3$ | $N(CH_3)_2$ | 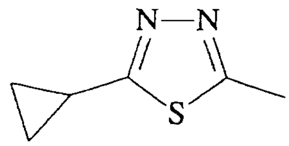 |
| $CH_3$ | $N(CH_3)_2$ | 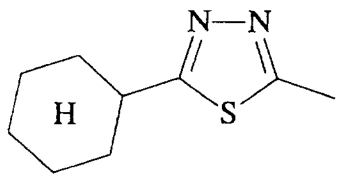 |
| $CH_3$ | $N(CH_3)_2$ | 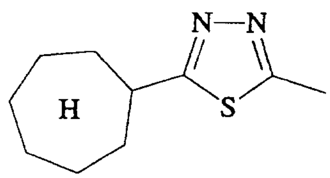 |
| $CH_3$ | $N(CH_3)_2$ | 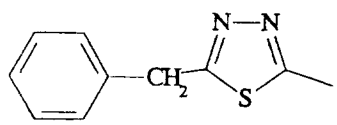 |
| $CH_3$ | $N(CH_3)_2$ | 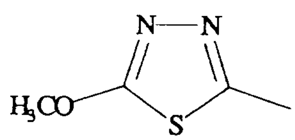 |
| $CH_3$ | $N(CH_3)_2$ | 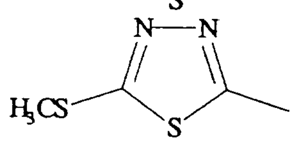 |
| $CH_3$ | $N(CH_3)_2$ | 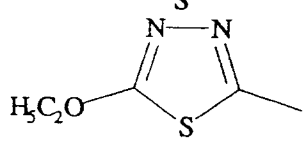 |

| $R^1$ | $R^2$ | Het |
|---|---|---|
| $CH_3$ | $N(CH_3)_2$ | 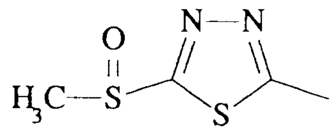 |
| $CH_3$ | $N(CH_3)_2$ | 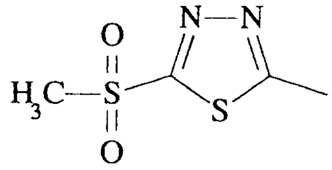 |
| $CH_3$ | $N(CH_3)_2$ | 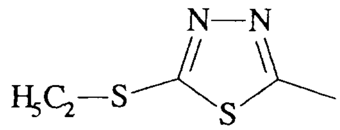 |
| $CH_3$ | $N(CH_3)_2$ | 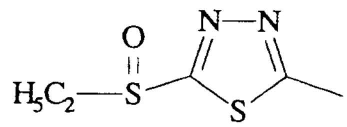 |
| $CH_3$ | $N(CH_3)_2$ | 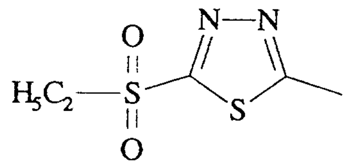 |
| $CH_3$ | $N(CH_3)_2$ | 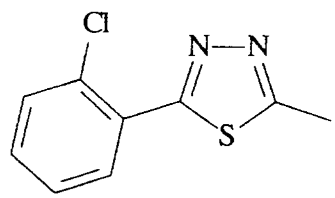 |
| $CH_3$ | $N(CH_3)_2$ | 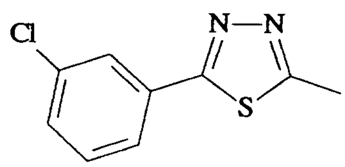 |

| $R^1$ | $R^2$ | Het |
|---|---|---|
| $CH_3$ | 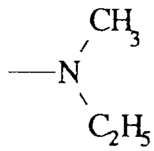 | 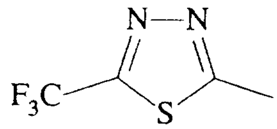 |
| $CH_3$ | $N(CH_3)_2$ | 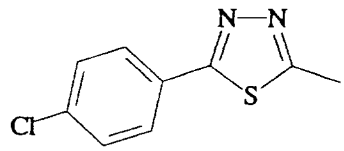 |
| $CH_3$ | 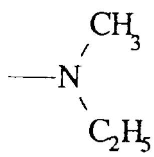 | 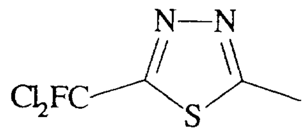 |
| $CH_3$ | $N(CH_3)_2$ | 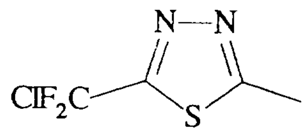 |
| $CH_3$ | $N(CH_3)_2$ | 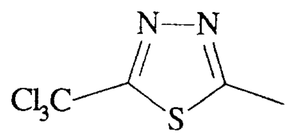 |
| $CH_3$ | $N(C_2H_5)_2$ | 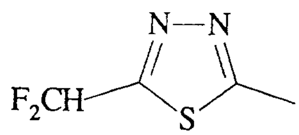 |
| $CH_3$ | $N(CH_3)_2$ | 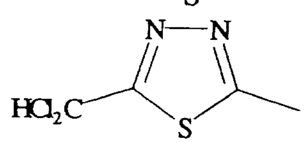 |
| $CH_3$ | $N(CH_3)_2$ | 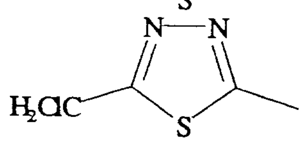 |
| $CH_3$ | $N(CH_3)_2$ | 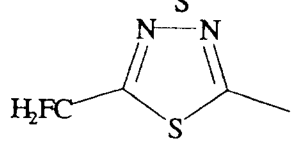 |

| $R^1$ | $R^2$ | Het |
|---|---|---|
| $C_2H_5$ | $N(CH_3)_2$ | 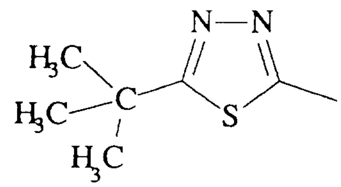 |
| $C_2H_5$ | 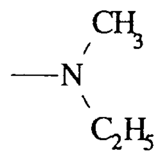 | 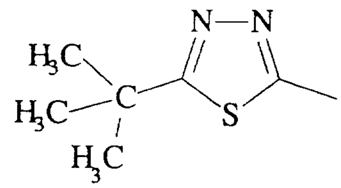 |
| $C_2H_5$ | $N(C_2H_5)_2$ | 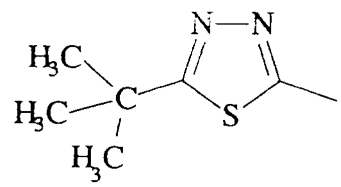 |
| $C_2H_5$ | 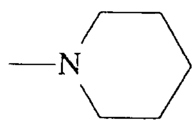 | 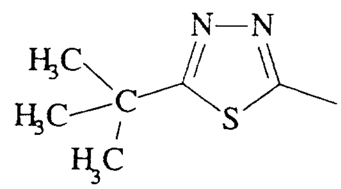 |
| $C_2H_5$ | 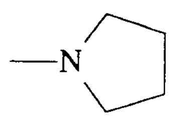 | 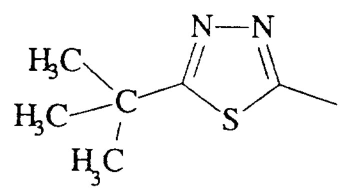 |
| $C_2H_5$ | 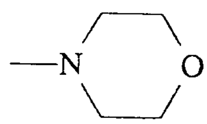 | 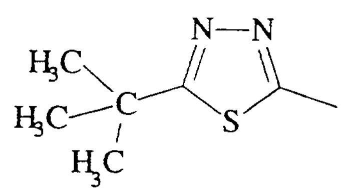 |
| 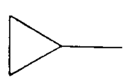 | $N(CH_3)_2$ | 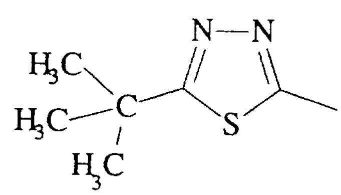 |

| $R^1$ | $R^2$ | Het |
|---|---|---|
| 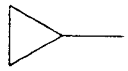 | 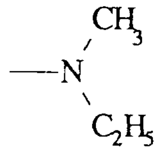 | 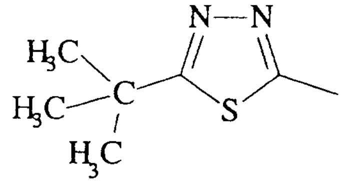 |
| 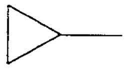 | $N(C_2H_5)_2$ | 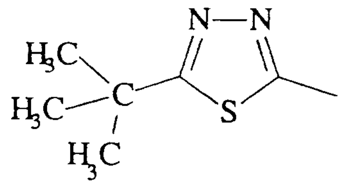 |
| 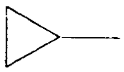 | 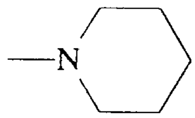 | 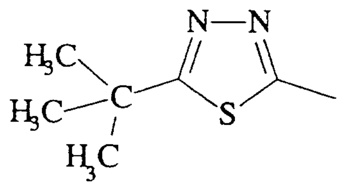 |
| 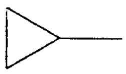 | 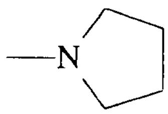 | 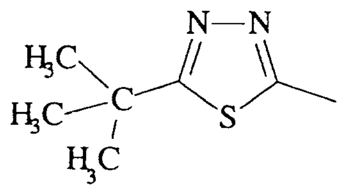 |
| 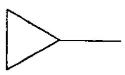 | 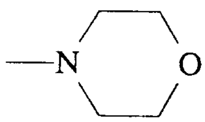 | 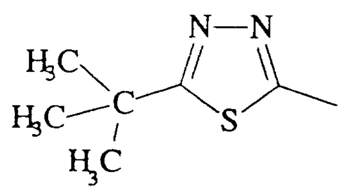 |
| $CH_3$ | $N(CH_3)_2$ | 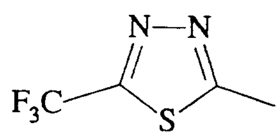 |
| $CH_3$ | 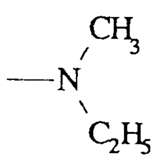 | 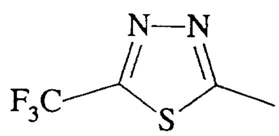 |

| $R^1$ | $R^2$ | Het |
|---|---|---|
| $CH_3$ | $N(C_2H_5)_2$ | 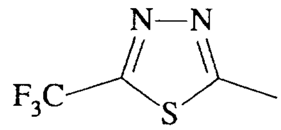 |
| $CH_3$ | 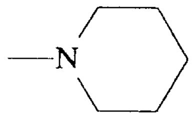 | 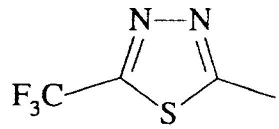 |
| $CH_3$ | 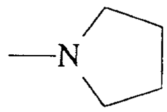 | 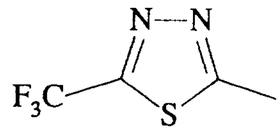 |
| $CH_3$ | 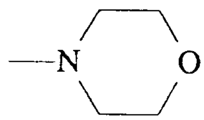 | 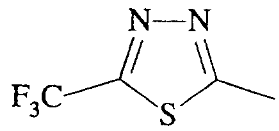 |
| $C_2H_5$ | $N(CH_3)_2$ | 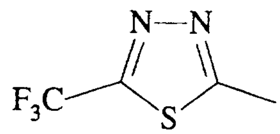 |
| $C_2H_5$ | 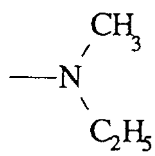 | 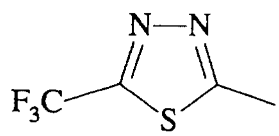 |
| $C_2H_5$ | $N(C_2H_5)_2$ | 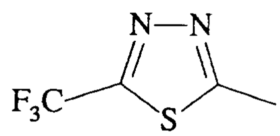 |
| $C_2H_5$ | 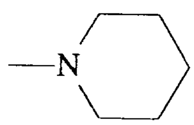 | 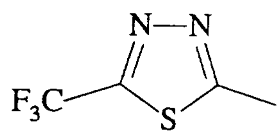 |
| $C_2H_5$ | 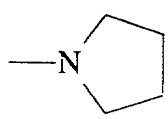 | 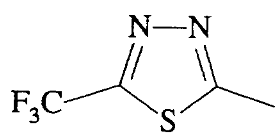 |

| $R^1$ | $R^2$ | Het |
|---|---|---|
| $C_2H_5$ | 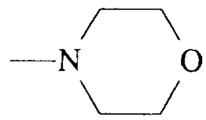 | 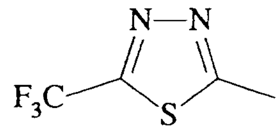 |
| $CH_3$ | 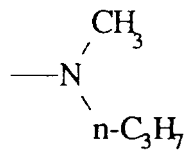 | 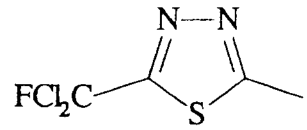 |
| $CH_3$ | $N(C_2H_5)_2$ | 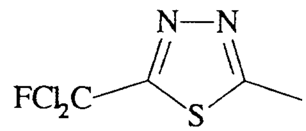 |
| $CH_3$ | 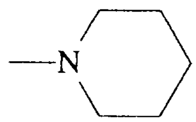 | 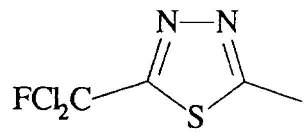 |
| $CH_3$ | 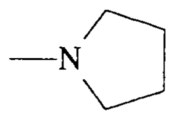 | 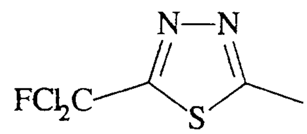 |
| $CH_3$ | 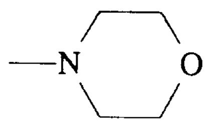 | 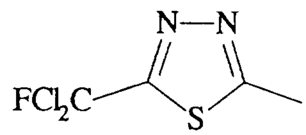 |
| $C_2H_5$ | $N(CH_3)_2$ | 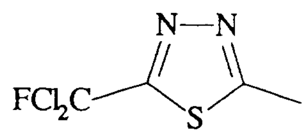 |
| $C_2H_5$ | $N(C_2H_5)_2$ | 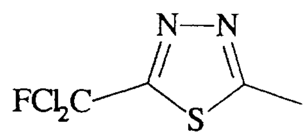 |
| $C_2H_5$ | 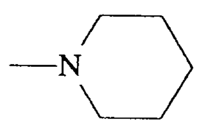 | 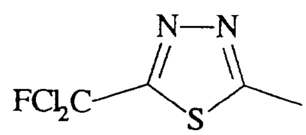 |
| $C_2H_5$ | 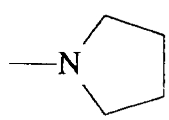 | 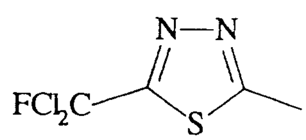 |

| $R^1$ | $R^2$ | Het |
|---|---|---|
| $C_2H_5$ | 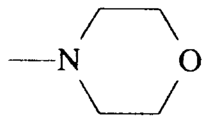 | 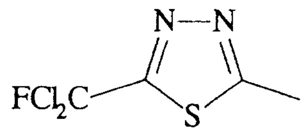 |
| $CH_3$ | 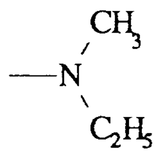 | 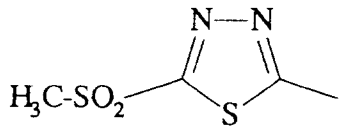 |
| $CH_3$ | 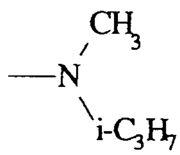 | 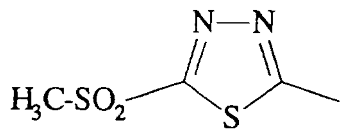 |
| $CH_3$ | $N(C_2H_5)_2$ | 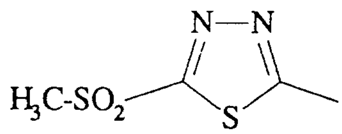 |
| $CH_3$ | 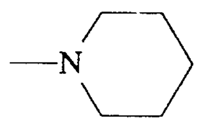 | 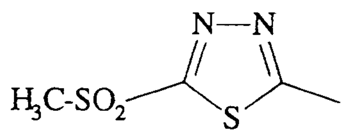 |
| $CH_3$ | 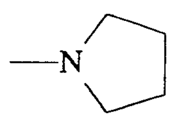 | 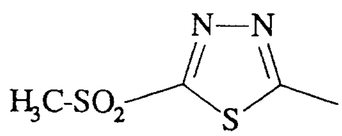 |
| $CH_3$ | 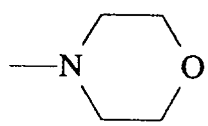 | 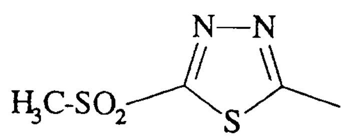 |

| $R^1$ | $R^2$ | Het |
|---|---|---|
| $C_2H_5$ | $N(CH_3)_2$ | 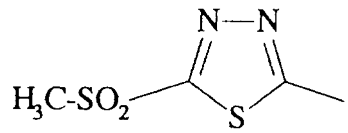 |
| $C_2H_5$ | 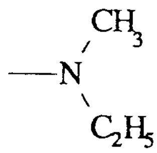 | 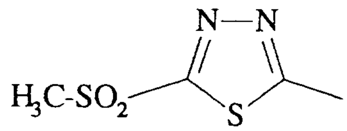 |
| $C_2H_5$ | $N(C_2H_5)_2$ | 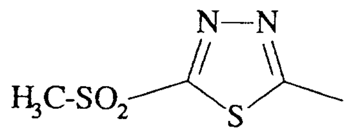 |
| $C_2H_5$ | 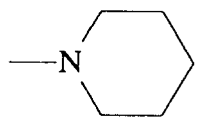 | 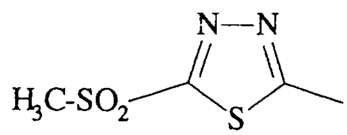 |
| $C_2H_5$ | 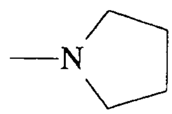 | 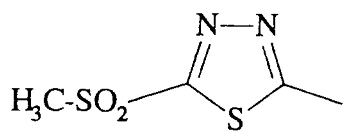 |
| $C_2H_5$ | 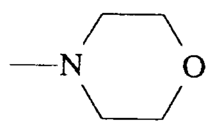 | 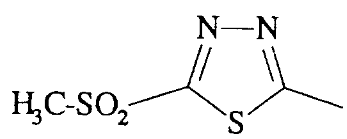 |
| $CH_3$ | $SCH_3$ | 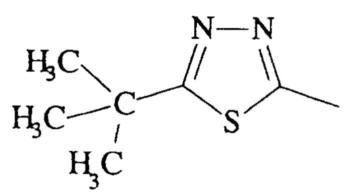 |

| $R^1$ | $R^2$ | Het |
|---|---|---|
| $CH_3$ | $SCH_3$ | 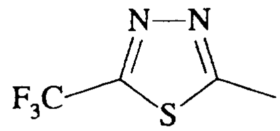 |
| $CH_3$ | $SCH_3$ | 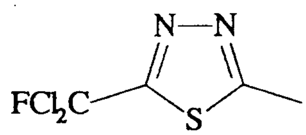 |
| $CH_3$ | $SCH_3$ | 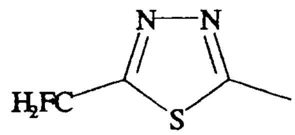 |
| $CH_3$ | $SCH_3$ | 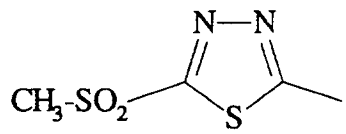 |
| $CH_3$ | $CH_3$ | 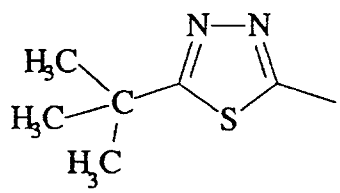 |
| $CH_3$ | $CH_3$ | 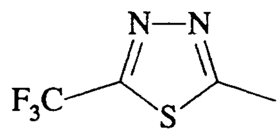 |
| $CH_3$ | $CH_3$ | 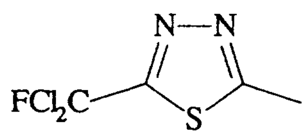 |
| $CH_3$ | $CH_3$ | 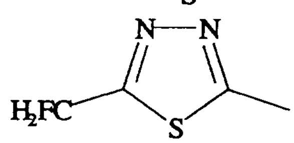 |

| $R^1$ | $R^2$ | Het |
|---|---|---|
| $CH_3$ | $CH_3$ | 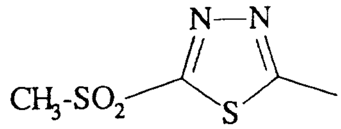 |
| $CH_3$ | $C_2H_5$ | 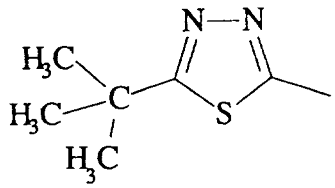 |
| $CH_3$ | $C_2H_5$ | 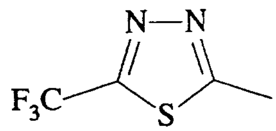 |
| $CH_3$ | $C_2H_5$ | 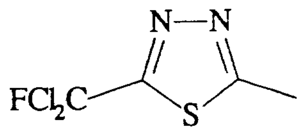 |
| $CH_3$ | $C_2H_5$ | 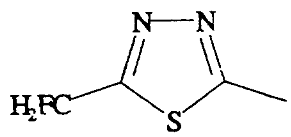 |
| $CH_3$ | $C_2H_5$ | 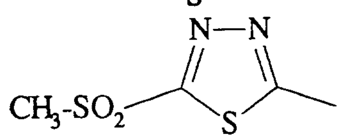 |
| $CH_3$ | 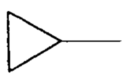 | 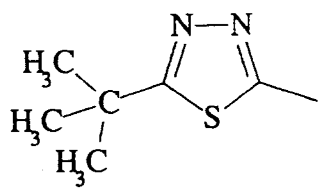 |
| $CH_3$ | 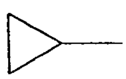 | 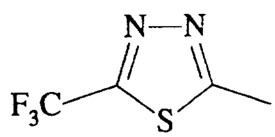 |

| $R^1$ | $R^2$ | Het |
|---|---|---|
| $CH_3$ | 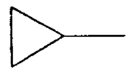 | 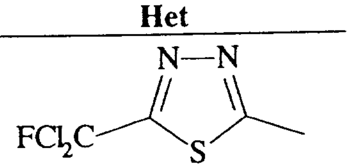 |
| $CH_3$ | 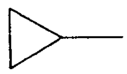 | 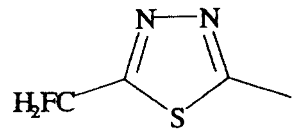 |
| $CH_3$ | 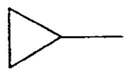 | 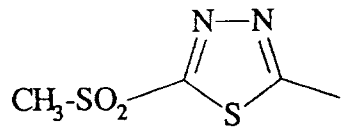 |
| $CH_3$ | $CH_3O$ | 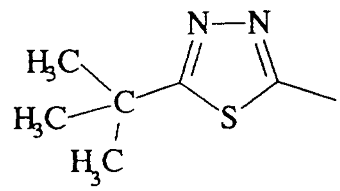 |
| $CH_3$ | $CH_3O$ | 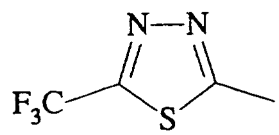 |
| $CH_3$ | $CH_3O$ | 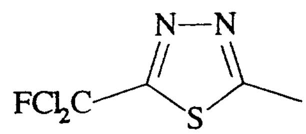 |
| $CH_3$ | $CH_3O$ | 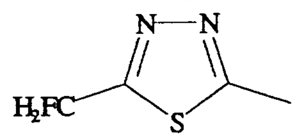 |
| $CH_3$ | $CH_3O$ | 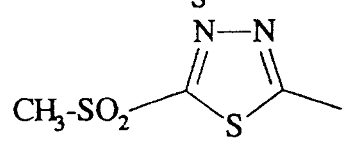 |

| $R^1$ | $R^2$ | Het |
|---|---|---|
| $CH_3$ | NH-$CH_3$ | 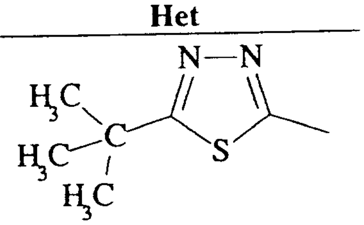 |
| $CH_3$ | NH-$CH_3$ | 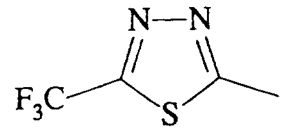 |
| $CH_3$ | NH-$CH_3$ | 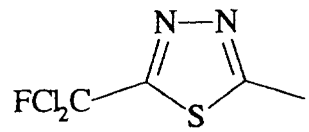 |
| $CH_3$ | NH-$CH_3$ | 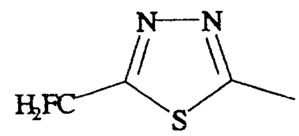 |
| $CH_3$ | NH-$CH_3$ | 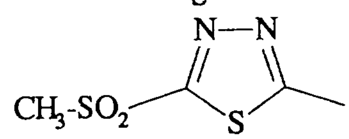 |
| $CH_3$ | $C_2H_5O$ | 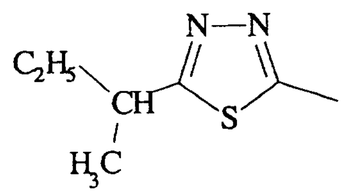 |
| $CH_3$ | $C_2H_5O$ | 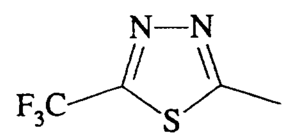 |
| $CH_3$ | $C_2H_5O$ | 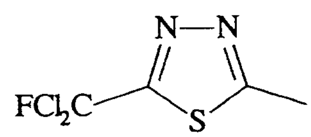 |
| $CH_3$ | $C_2H_5O$ | 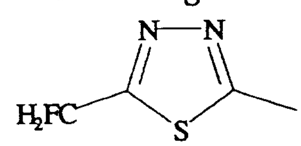 |

| $R^1$ | $R^2$ | Het |
|---|---|---|
| $CH_3$ | $C_2H_5O$ | 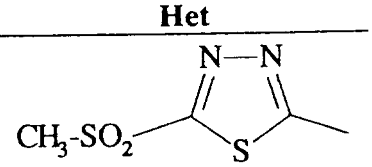 |
| $CH_3$ | $N(CH_3)_2$ | 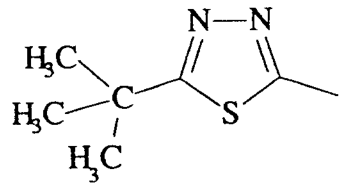 |
| $CH_3$ | $N(CH_3)_2$ | 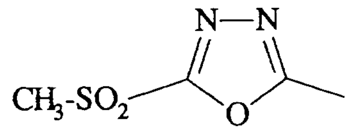 |
| $CH_3$ | $N(CH_3)_2$ | 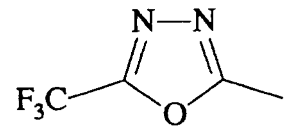 |
| $CH_3$ | $N(CH_3)_2$ | 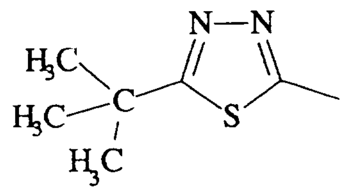 |
| $CH_3$ | $N(CH_3)_2$ | 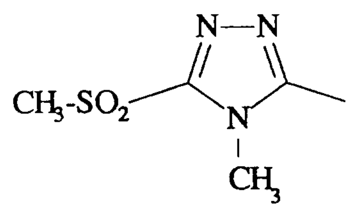 |
| $CH_3$ | $N(CH_3)_2$ | 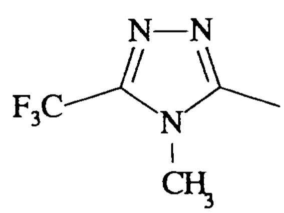 |

| $R^1$ | $R^2$ | Het |
|---|---|---|
| $CH_3$ | $N(CH_3)_2$ | 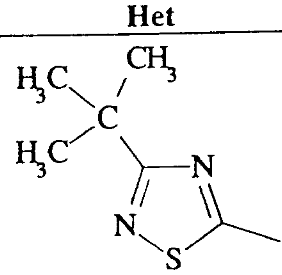 |
| $CH_3$ | $N(CH_3)_2$ | 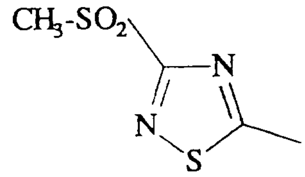 |
| $CH_3$ | $N(CH_3)_2$ | 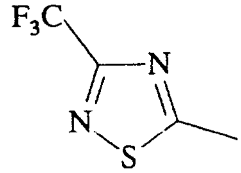 |
| $CH_3$ | $N(CH_3)_2$ | 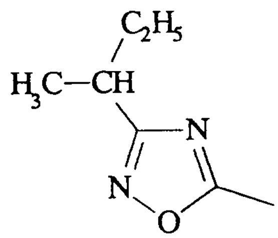 |
| $CH_3$ | $N(CH_3)_2$ | 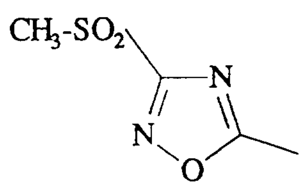 |
| $CH_3$ | $N(CH_3)_2$ | 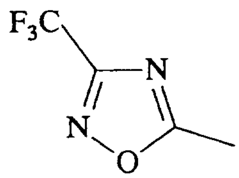 |

| $R^1$ | $R^2$ | Het |
|---|---|---|
| $CH_3$ | $N(CH_3)_2$ | 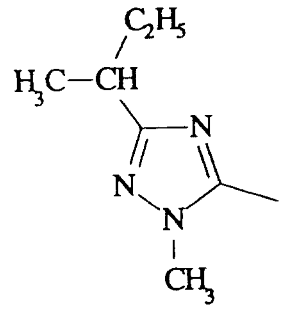 |
| $CH_3$ | $N(CH_3)_2$ | 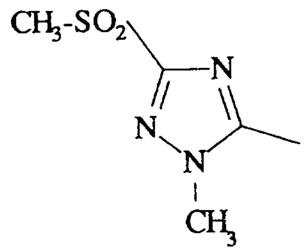 |
| $CH_3$ | $N(CH_3)_2$ | 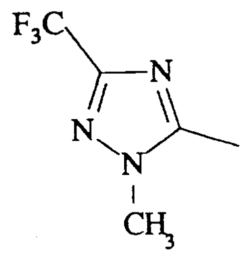 |
| $CH_3$ | $N(CH_3)_2$ | 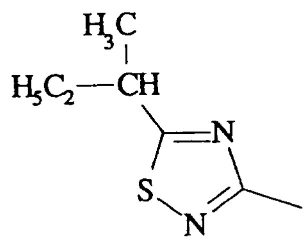 |
| $CH_3$ | $N(CH_3)_2$ | 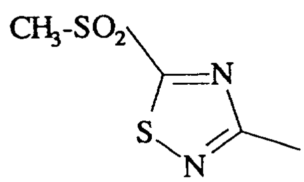 |

| $R^1$ | $R^2$ | Het |
|---|---|---|
| $CH_3$ | $N(CH_3)_2$ | 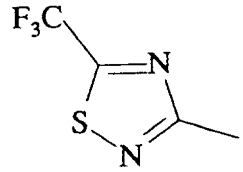 |
| $CH_3$ | $N(CH_3)_2$ | 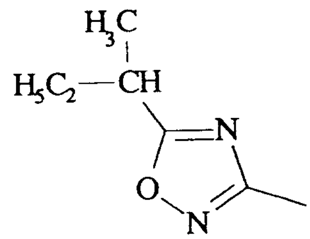 |
| $CH_3$ | $N(CH_3)_2$ | 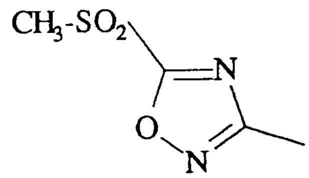 |
| $CH_3$ | $N(CH_3)_2$ | 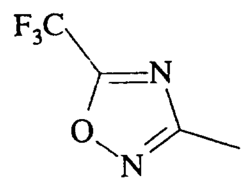 |
| $CH_3$ | $N(CH_3)_2$ | 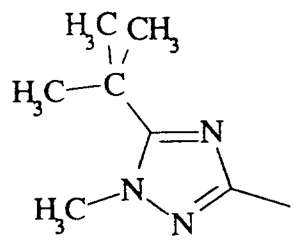 |
| $CH_3$ | $N(CH_3)_2$ | 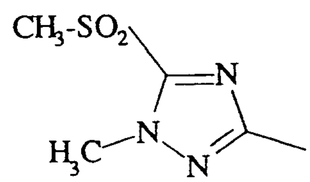 |
| $CH_3$ | $N(CH_3)_2$ | 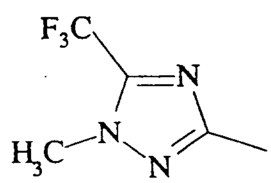 |

Verwendet man beispielsweise 2-Methyl-3-diethylamino-1,2,4-(1H)-triazolin-5-on und 2-Methylsulfonyl-5-t-butyl-1,3,4-thiadiazol als Ausgangsstoffe, so läßt sich der Reaktionsablauf des erfindungsgemäßen Verfahrens durch das folgende Formelschema darstellen:

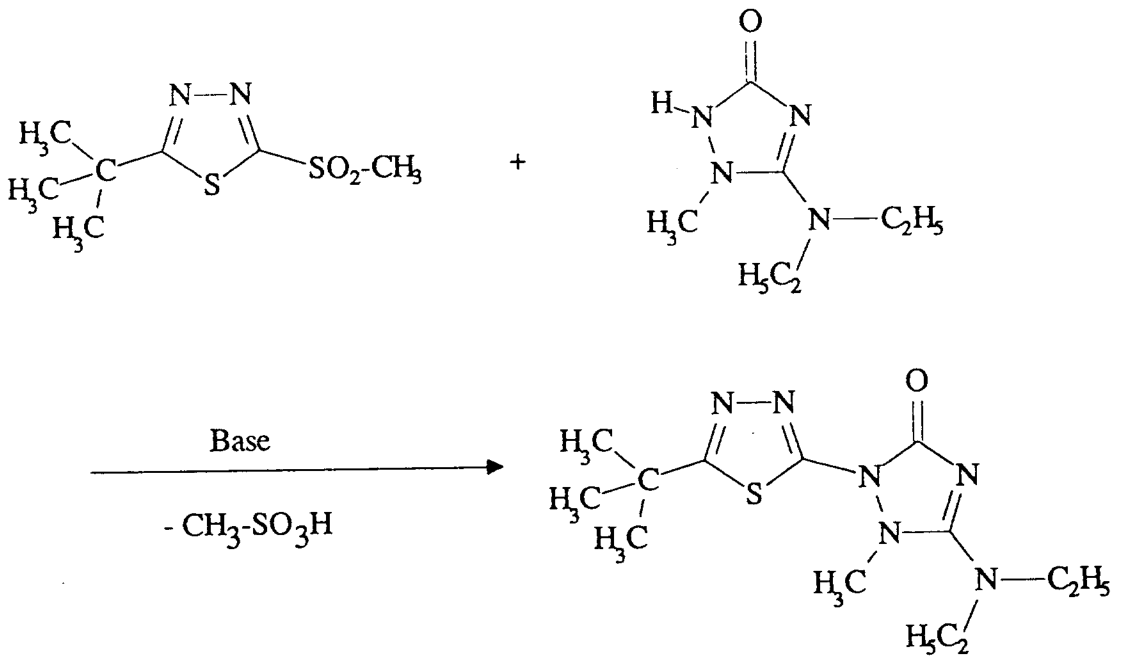


Die zur Durchführung des erfindungsgemäßen Verfahrens als Ausgangsstoffe benötigten Heterocyclen sind durch die Formel (II) allgemein definiert. In dieser Formel (II) steht Het vorzugsweise für diejenigen Reste, die bereits im Zusammenhang mit der Beschreibung der erfindungsgemäßen Verbindungen der Formel (I) als bevorzugt für diesen Substituenten genannt wurden.

E steht für einen üblichen Abgangsrest, vorzugsweise für Halogen, insbesondere für Chlor, Brom oder Iod oder für jeweils gegebenenfalls substituiertes Alkylsulfonyl, Alkylsulfonyloxy, Alkoxysulfonyloxy oder Arylsulfonyloxy, wie insbesondere Methansulfonyl, Ethansulfonyl, Methansulfonyloxy, Trifluormethansulfonyloxy, Methoxysulfonyloxy, Ethoxysulfonyloxy oder p-Toluolsulfonyloxy.

Die Heterocyclen der Formel (II) sind bekannt oder erhältlich in Analogie zu bekannten Verfahren (vergl. z.B. J. Med. Chem. 31, 906 [1988]; US 4.454.147; US 3.959.301; J.Prakt. Chem.315, 611, [1973]; DE 40 31 158; DE 40 03 436).

Die zur Durchführung des erfindungsgemäßen Verfahrens weiterhin als Ausgangsstoffe benötigten Triazolinone sind durch die Formel (III) allgemein definiert. In dieser Formel (III) stehen $R^1$ und $R^2$ vorzugsweise für diejenigen Reste, die bereits im Zusammenhang mit der Beschreibung der erfindungsgemäßen Verbindungen der Formel (I) als bevorzugt für diese Substituenten genannt wurden.

Die Triazolinone der Formel (III) sind bekannt oder erhältlich in Analogie zu bekannten Verfahren (vergl. z.B. DE 39 39 952; Bull. Chim. Soc. Fr. 1975, 1191; DE 24 07 304; DE 25 37 973; DE 24 23 765)

Als Verdünnungsmittel zur Durchführung des erfindungsgemäßen Verfahrens kommen inerte organische Lösungsmittel infrage. Hierzu gehören insbesondere aliphatische, alicyclische oder aromatische, gegebenenfalls halogenierte Kohlenwasserstoffe, wie beispielsweise Benzin, Benzol, Toluol, Xylol, Chlorbenzol, Dichlorbenzol, Petrolether, Hexan, Cyclohexan, Dichlormethan, Chloroform, Tetrachlorkohlenstoff; Ether, wie Diethylether, Diisopropylether, Dioxan, Tetrahydrofuran oder Ethylenglykoldimethyl-oder -diethylether, Ketone, wie Aceton, Butanon oder Methyl-isobutylketon; Nitrile, wie Acetonitril, Propionitril oder Benzonitril; Amide, wie N,N-Dimethylformamid, N,N-Dimethylacetamid, N-Methylformanilid, N-Methylpyrrolidon oder Hexamethylphosphorsäuretriamid; Ester wie Essigsäuremethylester oder Essigsäureethylester oder Sulfoxide, wie Dimethylsulfoxid.

Das erfindungsgemäße Verfahren kann gegebenenfalls in Gegenwart eines geeigneten basischen Reaktionshilfsmittels durchgeführt werden. Als solche kommen alle üblichen anorganischen oder organischen Basen infrage. Hierzu gehören beispielsweise Erdalkali-oder Alkalimetallhydroxide, wie Natriumhydroxid, Calciumhydroxid, Kaliumhydroxid oder auch Ammoniumhydroxid, Alkalimetallcarbonate, wie Natriumcarbonat, Kaliumcarbonat, Kaliumhydrogencarbonat, Natriumhydrogencarbonat oder Ammoniumcarbonat, Alkali- oder Erdalkalimetallacetate, wie Natriumacetat, Kaliumacetat, Calciumacetat oder Ammoniumacetat, sowie tertiäre Amine, wie Trimethylamin, Triethylamin, Tributylamin, N,N-Dimethylanilin, Pyridin, N-Methylpiperidin, N,N-Dimethylaminopyridin, Diazabicyclooctan (DABCO), Diazabicyclononen (DBN) oder Diazabicycloundecen (DBU).

Das erfindungsgemäße Verfahren kann gegebenenfalls auch in einem Zweiphasensystem, wie beispielsweise Wasser/Toluol oder Wasser/Dichlormethan, gegebenenfalls in Gegenwart eines geeigneten Phasentransferkatalysators, durchgeführt werden. Als Beispiele für solche Katalysatoren seien genannt: Tetrabutylammoniumiodid, Tetrabutylammoniumbromid, Tetrabutylammoniumchlorid, Tributyl-methylphosphoniumbromid, Trimethyl-$C_{13}/C_{15}$-alkylammoniumchlorid, Trimethyl-$C_{13}/C_{15}$-alkylammoniumbromid, Dibenzyl-dimethyl-ammoniummethylsulfat, Dimethyl-$C_{12}/C_{14}$-alkyl-benzylammoniumchlorid, Dimethyl-$C_{12}/C_{14}$-alkyl-benzylammoniumbromid, Tetrabutylammoniumhydroxid, Triethylbenzylammoniumchlorid, Methyltrioctylammoniumchlorid, Trimethylbenzylammoniumchlorid, 15-Krone-5, 18-Krone-6 oder Tris-[2-(2-methoxyethoxy)-ethyl]-amin.

Die Reaktionstemperaturen können bei der Durchführung des erfindungsgemäßen Verfahrens in einem größeren Bereich variiert werden. Im allgemeinen arbeitet man bei Temperaturen zwischen -20 °C und +180 °C, vorzugsweise bei Temperaturen zwischen 20 °C und 150 °C.

Das erfindungsgemäße Verfahren wird üblicherweise unter Normaldruck durchgeführt. Es ist jedoch auch möglich unter erhöhtem oder vermindertem Druck zu arbeiten.

Zur Durchführung des erfindungsgemäßen Verfahrens setzt man pro Mol Heterocyclus der Formel (II) im allgemeinen 1,0 bis 3,0 Mol, vorzugsweise 1,0 bis 1,5 Mol Triazolinon der Formel (III) und 1,0 bis 5,0 Mol, vorzugsweise 1,0 bis 2,5 Mol Base als Reaktionshilfsmittel und gegebenenfalls 0,001 bis 2,0 Mol, vorzugsweise 0,001 bis 1,0 Mol Phasentransferkatalysator ein.

Die Reaktionsdurchführung, Aufarbeitung und Isolierung der Reaktionsprodukte erfolgt nach bekannten Verfahren (vergl. hierzu auch die Herstellungsbeispiele).

Die Reinigung der Endprodukte der Formel (I) erfolgt mit Hilfe üblicher Verfahren, beispielsweise durch Säulenchromatographie oder durch Umkristallisieren.

Die Charakterisierung erfolgt mit Hilfe des Schmelzpunktes oder bei nicht kristallisierenden Verbindungen mit Hilfe des Brechungsindex oder der Protonen-Kernresonanzspektroskopie ($^1$H-NMR).

Die erfindungsgemäßen Wirkstoffe können als Defoliants, Desiccants, Krautabtötungsmittel und insbesondere als Unkrautvernichtungsmittel verwendet werden.Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:

Dikotyle Unkräuter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea.

Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen, z.B. Forst, Ziergehölz-, Obst-, Wein-, Zitrus-, Nuß-, Bananen-, Kaffee-, Tee-, Gummi-, Ölpalm-, Kakao-, Beerenfrucht- und Hopfenanlagen und zur selektiven Unkrautbekämpfung in einjährigen Kulturen eingesetzt werden.

Dabei lassen sich die erfindungsgemäßen Wirkstoffe mit besonders gutem Erfolg zur Bekämpfung von mono- und dikotylen Unkräutern in monokotylen und dikotylen Kulturen wie beispielsweise Weizen, Mais oder Soja einsetzen.

Die Wirkstoffe können in die üblichen Formulierungen übergeführt werden, wie Lösungen, Emulsionen, Suspensionen, Pulver, Schäume, Pasten, Granulate, Aerosole, Wirkstoff-imprägnierte Natur- und synthetische Stoffe, Feinstverkapselungen in polymeren Stoffen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen infrage: Aromaten, wie Xylol, Toluol, Alkylnaphthaline, chlorierte Aromaten oder chlorierte aliphatische Kohlenwasserstoffe, wie Chlorbenzole, Chlorethylene, oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone, wie Aceton, Methylethylketon, Methylisobutylketon oder Cyclohexanon, stark polare Lösungsmittel, wie Dimethylformamid oder Dimethylsulfoxid, sowie Wasser.

Als feste Trägerstoffe kommen infrage: z.B. Ammoniumsalze und natürliche Gesteinsmehle, wie Kaoline, Tonerden, Talkum, Kreide, Quartz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen infrage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier- und/oder schaumerzeugende Mittel kommen infrage: z.B. nicht ionogene und anionische Emulgatoren, wie Polyoxyethylen-Fettsäureester, Polyoxyethylen-Fettalkohol-Ether, z.B. Alkylarylpolyglykolether, Alkylsulfonate, Alkylsulfate, Arylsulfonate sowie Eiweißhydrolysate; als Dispergiermittel kommen infrage: z.B. Ligninsulfitablaugen und Methylcellulose.

Es können in den Formulierungen Haftmittel wie Carboxymethylcellulose, natürliche und synthetische, pulverige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid,Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinenzwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide infrage, beispielsweise Anilide, wie z.B. Diflufenican und Propanil; Arylcarbonsäuren, wie z.B. Dichlorpicolinsäure, Dicamba oder Picloram; Aryloxyalkansäuren, wie z.B. 2,4-D, 2,4-DB, 2,4-DP, Fluroxypyr, MCPA, MCPP und Triclopyr; Aryloxy-phenoxy-alkansäureester, wie z.B. Diclofop-methyl, Fenoxaprop-ethyl, Fluazifop-butyl, Haloxyfop-methyl und Quizalofopethyl; Azinone, wie z.B. Chloridazon und Nofflurazon; Carbamate, wie z.B. Chlorpropham, Desmedipham, Phenmedipham und Propham; Chloracetanilide, wie z.B. Alachlor, Acetochlor, Butachlor, Metazachlor, Metolachlor, Pretilachlor und Propachlor; Dinitroaniline, wie z.B. Oryzalin, Pendimethalin und Trifluralin; Diphenylether, wie :z.B. Acifluorfen, Bifenox, Fluoroglycofen, Fomesafen, Halosafen, Lactofen und Oxyfluorfen; Harnstoffe, wie z.B. Chlortoluron, Diuron, Fluometuron, Isoproturon, Linuron und Methabenzthiazuron; Hydroxylamine, wie z.B. Alloxydim, Clethodim, Cycloxydim, Sethoxydim und Trtlkoxydim; Imidazolinone, wie z.B. Imazethapyr, Imazamethabenz, Imazapyr und Imazaquin; Nitrile, wie z.B. Bromoxynil, Dichlobenil und Ioxynil; Oxyacetamide, wie z.B. Mefenacet; Sulfonylharnstoffe, wie z.B. Amidosulfuron, Bensulfuron-methyl, Chlorimuron-ethyl, Chlorsulfuron, Cinosulfuron, Metsulfuron-methyl, Nicosulfuron, Primisulfuron, Pyrazosulfuron-ethyl, Thifensulfuron-methyl, Triasulfuron und Tribenuron-methyl; Thiolcarbamate, wie z.B. Butylate, Cycloate, Diallate, EPTC, Esprocarb, Molinate, Prosulfocarb, Thiobencarb und Triallate; Triazine, wie z.B. Atrazin, Cyanazin, Simazin, Simetryne, Terbutryne und Terbutylazin; Triazinone, wie z.B. Hexazinon, Metamitron und Metribuzin; Sonstige, wie z.B. Aminotriazol, Benfuresate, Bentazone, Cinmethylin, Clomazone, Clopyralid, Difenzoquat, Dithiopyr, Ethofumesate, Fluorochloridone, Glufosinate, Glyphosate, Isoxaben, Pyridate, Quinchlorac, Quinmerac, Sulphosate und Tridiphane.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen; Sprühen, Streuen.

Die erfindungsgemäßen Wirkstoffe können sowohl vor als auch nach dem Auflaufen der Pflanzen appliziert werden.

Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 0,001 und 10 kg Wirkstoff pro Hektar Bodenfläche, vorzugsweise zwischen 0,005 und 5 kg pro Hektar.

Die Herstellung und die Verwendung der erfindungsgemäßen Wirkstoffe geht aus den nachfolgenden Beispielen hervor.

Herstellungsbeispiele:

Beispiel 1:

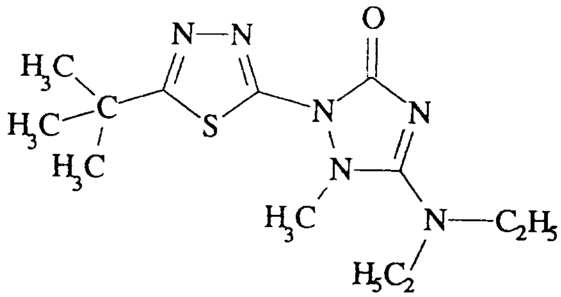

Zu 1,7 g (0,01 Mol) 3-Diethylamino-2-methyl-1,2,4-(1H)-triazolin-5-on (Herstellung analog DE 39 39 952) und 2,2 g (0,01 Mol) 2-Methylsulfonyl-5-t-butyl-1,3,4-thiadiazol (Herstellung analog DE 40 03 436 oder DE 34 22 861) in 50 ml Dimethylformamid gibt man 2,76 g (0,02 Mol) Kaliumcarbonat und erhitzt die Mischung für 20 Stunden auf 90 °C. Zur Aufarbeitung wird die abgekühlte Reaktionsmischung in Wasser gegossen, mit verdünnter Salzsäure angesäuert und zweimal mit Dichlormethan extrahiert. Die vereinigten organischen Phasen werden dreimal mit Wasser gewaschen, über Magnesiumsulfat getrocknet und im Vakuum eingeengt. Der Rückstand wird durch Überschichten mit Ether/Hexan (1:1) zur Kristallisation gebracht und abgesaugt.

Man erhält 1,1 g (35 % der Theorie) 1-(5-t-Butyl-1,3,4-thiadiazol-2-yl)-3-diethylamino-2-methyl-1,2,4-(1H)-triazolin-5-on mit Schmelzpunkt 93 °C.

In entsprechender Weise und gemäß den allgemeinen Angaben zur Herstellung erhält man die folgenden Heterocyclyltriazolinone der allgemeinen Formel (I):

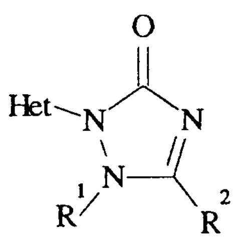

(I)

| Bsp.Nr. | $R^1$ | $R^2$ | Het | physikalische Eigenschaften |
|---|---|---|---|---|
| 2 | $CH_3$ | $N(CH_3)_2$ | 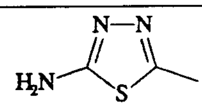 | Fp. 208°C |
| 3 | $CH_3$ | $N(CH_3)_2$ | 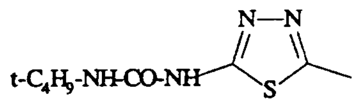 | Fp. >250°C |
| 4 | $CH_3$ | $N(CH_3)_2$ | 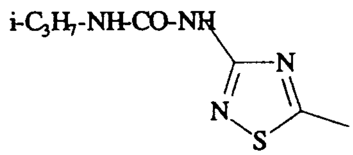 | Fp. >260°C |
| 5 | $CH_3$ | $N(CH_3)_2$ | 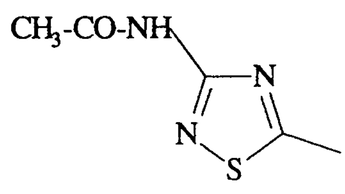 | Fp. >260°C |
| 6 | $CH_3$ | $N(CH_3)_2$ | 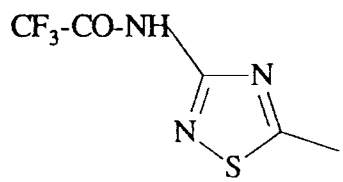 | Fp. >260°C |

| Bsp.Nr. | $R^1$ | $R^2$ | Het | physikalische Eigenschaften |
|---|---|---|---|---|
| 7 | $CH_3$ | $N(CH_3)_2$ | 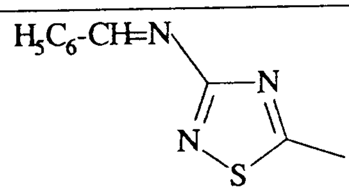 | Fp. 206°C |
| 8 | $CH_3$ | $N(CH_3)_2$ | 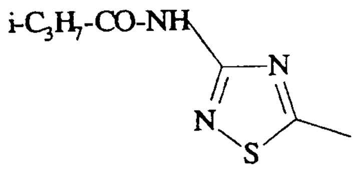 | Fp. 252°C |
| 9 | $CH_3$ | $N(CH_3)_2$ | 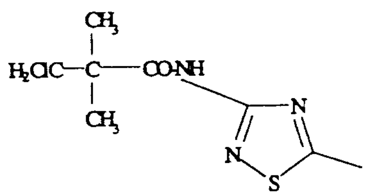 | Fp. 218°C |
| 10 | $CH_3$ | $N(CH_3)_2$ | 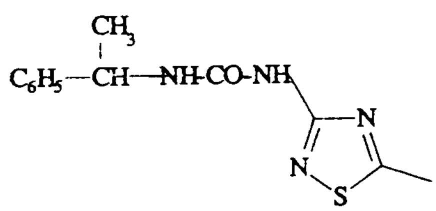 | Fp. >260°C |
| 11 | $CH_3$ | $N(CH_3)_2$ | 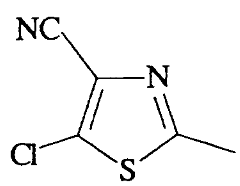 | Fp. 174°C |
| 12 | $CH_3$ | $N(C_2H_5)_2$ | 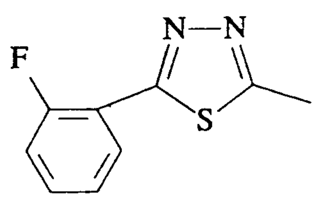 | Fp. 151°C |

| Bsp.Nr. | $R^1$ | $R^2$ | Het | physikalische Eigenschaften |
|---|---|---|---|---|
| 13 | $CH_3$ | $N(C_2H_5)_2$ | 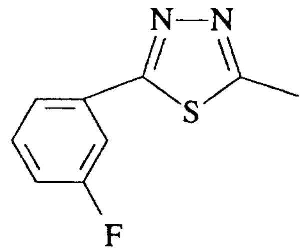 | Fp. 110°C |
| 14 | $CH_3$ | 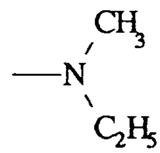 | 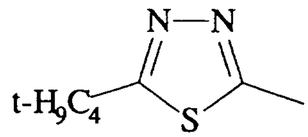 | Fp. 68°C |
| 15 | $CH_3$ | 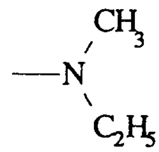 | 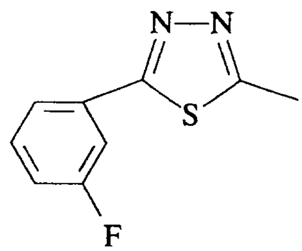 | Fp. 140°C |
| 16 | $CH_3$ | 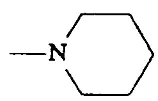 | 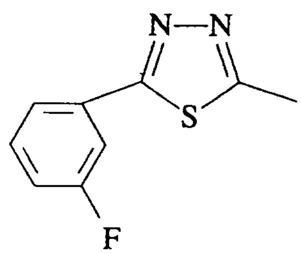 | Fp. 197°C |
| 17 | $CH_3$ | 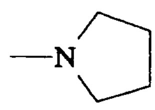 | 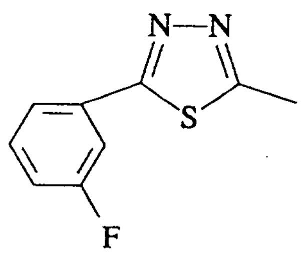 | Fp. 224°C |

| Bsp.Nr. | $R^1$ | $R^2$ | Het | physikalische Eigenschaften |
|---|---|---|---|---|
| 18 | $CH_3$ | $N(C_2H_5)_2$ | 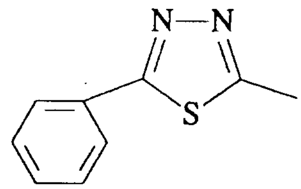 | Fp. 102°C |
| 19 | $CH_3$ | 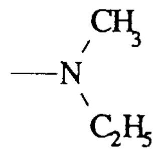 | 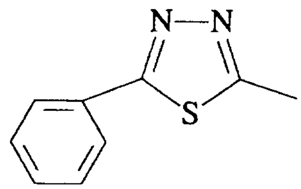 | Fp. 127°C |
| 20 | $CH_3$ | 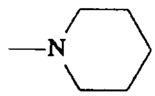 | 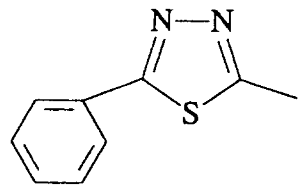 | Fp. 208°C |
| 21 | $CH_3$ | 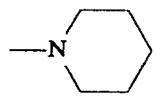 | 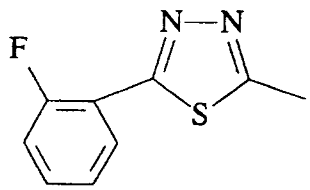 | Fp. 201°C |
| 22 | $CH_3$ | 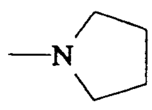 | 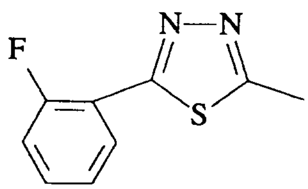 | Fp. 241°C |
| 23 | $CH_3$ | 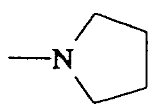 | 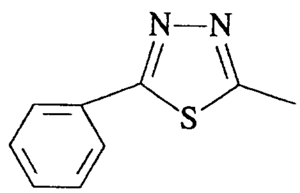 | Fp. 207°C |

| Bsp.Nr. | $R^1$ | $R^2$ | Het | physikalische Eigenschaften |
|---|---|---|---|---|
| 24 | $CH_3$ | 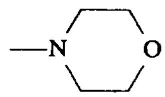 | 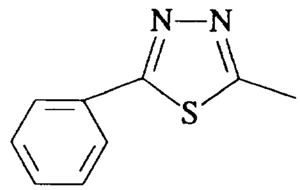 | Fp. 265°C |
| 25 | $CH_3$ | 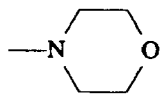 | 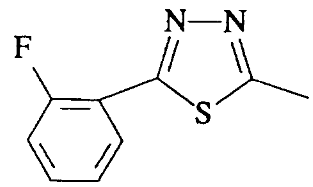 | Fp. 267°C |
| 26 | $CH_3$ | 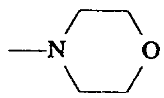 | 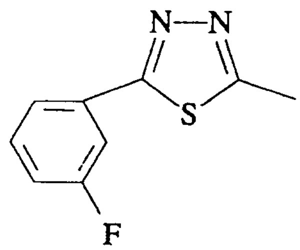 | Fp. 269°C |
| 27 | $CH_3$ | 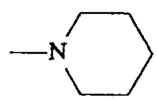 | 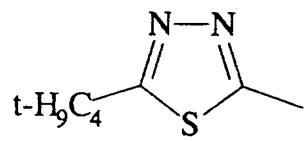 | Fp. 185°C |
| 28 | $CH_3$ | 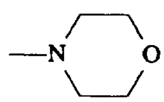 | 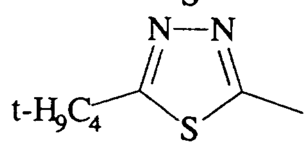 | Fp. 228°C |
| 29 | $CH_3$ | 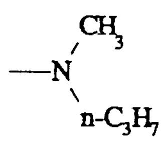 | 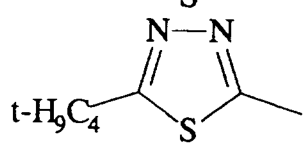 | Fp. 46°C |
| 30 | $CH_3$ | $-N(i-C_3H_7)_2$ | 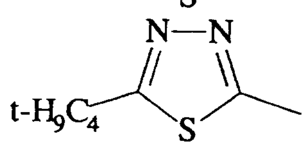 | Fp. 155°C |
| 31 | $CH_3$ | 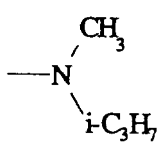 | 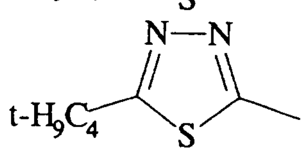 | Fp. 32°C |

| Bsp.Nr. | $R^1$ | $R^2$ | Het | physikalische Eigenschaften |
|---|---|---|---|---|
| 32 | $CH_3$ | 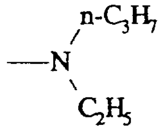 | 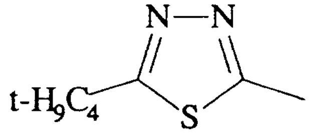 | Fp. 82°C |
| 33 | $CH_3$ | 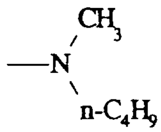 | 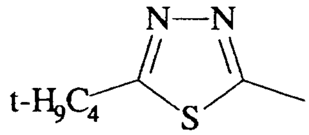 | Fp. 59°C |
| 34 | $CH_3$ | $N(CH_3)_2$ | 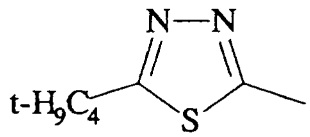 | $n_D^{20}$ = 1,5075 |
| 35 | $CH_3$ | $N(CH_3)_2$ | 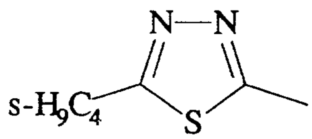 | $n_D^{20}$ = 1,5165 |
| 36 | $CH_3$ | 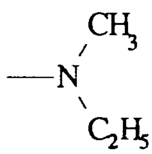 | 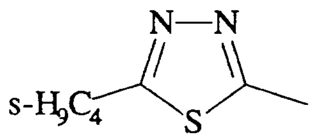 | $n_D^{20}$ = 1,5040 |
| 37 | $CH_3$ | $N(CH_3)_2$ | 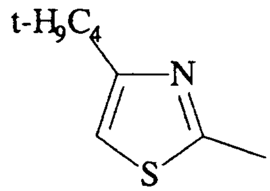 | Fp. 60°C |
| 38 | $CH_3$ | 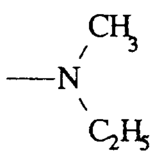 | 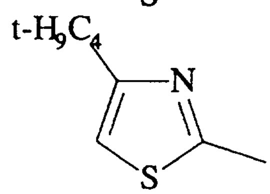 | Fp. 70°C |
| 39 | $CH_3$ | $N(CH_3)_2$ | 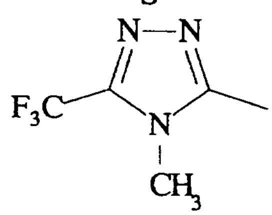 | Fp. 63°C |
| 40 | $CH_3$ | $N(CH_3)_2$ | 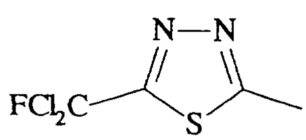 | Fp. 115°C |

| Bsp.Nr. | $R^1$ | $R^2$ | Het | physikalische Eigenschaften |
|---|---|---|---|---|
| 41 | $CH_3$ | $N(CH_3)_2$ | 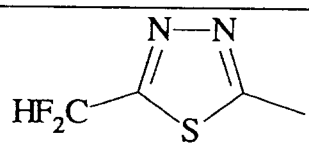 | Fp. 137°C |
| 42 | $CH_3$ | $N(CH_3)_2$ | 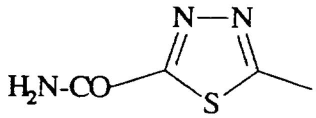 | Fp. >300°C |
| 43 | $CH_3$ | 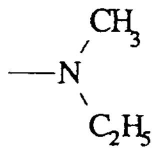 | 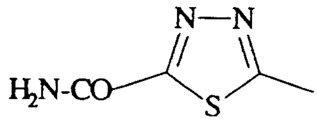 | Fp. 190°C |
| 44 | $CH_3$ | $N(CH_3)_2$ | 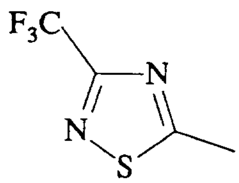 | Fp. 190°C |
| 45 | $CH_3$ | 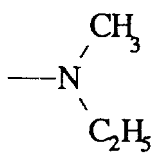 | 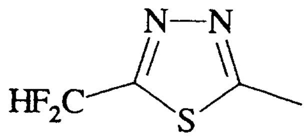 | Wachs |
| 46 | $CH_3$ | $N(CH_3)_2$ | 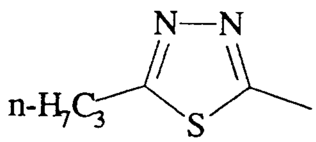 | Fp. 63°C |
| 47 | $CH_3$ | $N(CH_3)_2$ | 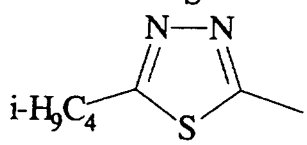 | Fp. 98°C |
| 48 | $CH_3$ | $N(CH_3)_2$ | 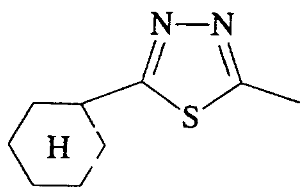 | Fp. 85°C |
| 49 | $CH_3$ | $N(CH_3)_2$ | 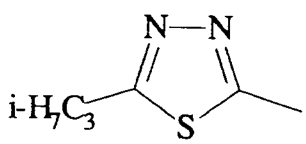 | Fp. 57°C |

| Bsp.Nr. | $R^1$ | $R^2$ | Het | physikalische Eigenschaften |
|---|---|---|---|---|
| 50 | $CH_3$ | $N(CH_3)_2$ | 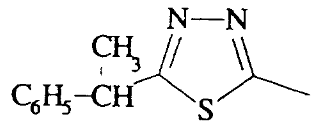 | Wachs |
| 51 | $CH_3$ | $N(CH_3)_2$ | 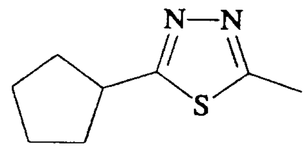 | Fp. 65°C |
| 52 | $CH_3$ | $N(CH_3)_2$ | 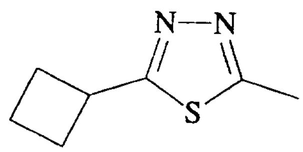 | Fp. 105°C |
| 53 | $CH_3$ | $N(CH_3)_2$ | 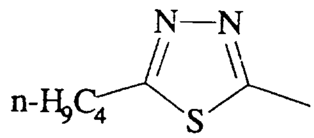 | Fp. 40°C |
| 54 | $CH_3$ | $N(CH_3)_2$ | 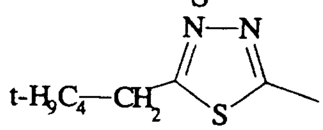 | Fp. 127°C |
| 55 | $CH_3$ | $CH_3$ | 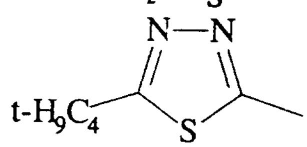 | Fp. 45°C |

Anwendungsbeispiele:

In den folgenden Anwendungsbeispielen wurde die nachstehend aufgeführte Verbindung als Vergleichssubstanz eingesetzt:

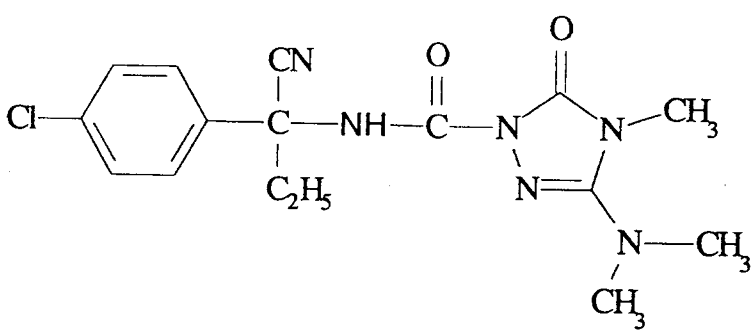

(A)

1-{1-[1-(4-Chlorphenyl)-1-cyano]-propyl-aminocarbonyl]}-3-dimethylamino-4-methyl-1,2,4-triazolin-5-on (bekannt aus EP 283 876)

Beispiel A:

**Pre-emergence-Test**

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man ein Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoffzubereitung begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffes pro Flächeneinheit. Nach drei Wochen wird der Schädigungsgrad der Pflanzen bonitiert in % Schädigung im Vergleich zur Entwicklung der unbehandelten Kontrolle. Es bedeuten:

0 % = keine Wirkung (wie unbehandelte Kontrolle)
100 % = totale Vernichtung

Eine deutliche Überlegenheit in der Wirksamkeit bei vergleichbarer Nutzpflanzenselektivität gegenüber dem Stand der Technik zeigen in diesem Test z.B. die Verbindungen gemäß den Herstellungsbeispielen 1, 14, 31, 41 und 45.

Beipiel B:

**Post-emergence-Test**

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man ein Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5 bis 15 cm haben so, daß die jeweils gewünschten Wirkstoffinengen pro Flächeneinheit ausgebracht werden. Nach drei Wochen wird der Schädigungsgrad der Pflanzen bonitiert in % Schädigung im Vergleich zur Entwicklung der unbehandelten Kontrolle.

Es bedeuten:

0 % = keine Wirkung (wie unbehandelte Kontrolle)
100% = totale Vernichtung

Eine deutliche Überlegenheit in der Wirksamkeit ebenso wie in der Nutzpflanzenselektivität gegenüber dem Stand der Technik zeigen in diesem Test z.B. die Verbindungen gemäß den Herstellungsbeispielen 1, 13, 14, 28, 29, 31, 34 und 41.

**Patentansprüche**

1. Heterocyclyltriazolinone der allgemeinen Formel (I)

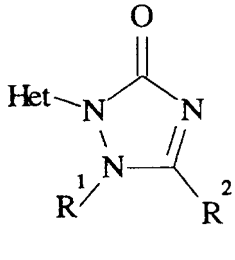

(I)

in welcher

$R^1$ für Wasserstoff oder für jeweils gegebenenfalls substituiertes Alkyl oder Cycloalkyl steht,

$R^2$ für Wasserstoff, Hydroxy, Mercapto, Halogen oder für jeweils gegebenenfalls substituiertes Alkyl, Alkenyl, Alkinyl, Cycloalkyl, Alkoxy, Alkylthio, Alkylsulfinyl, Alkylsulfonyl, Arylalkyl, Amino oder Heterocyclyl steht und

Het für einen gegebenenfalls substituierten Heterocyclus der Formel

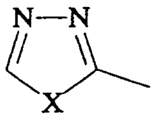 ; 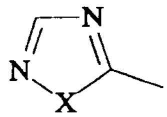 ; 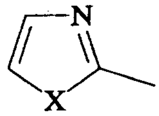 oder 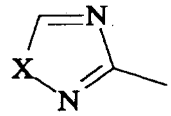

steht, wobei

X jeweils für Sauerstoff, Schwefel, eine NH-Gruppe oder eine N-Alkylgruppe steht.

**2.** Heterocyclyltriazolinone der allgemeinen Formel (I) gemäß Anspruch 1, dadurch gekennzeichnet, daß

$R^1$ für Wasserstoff oder für gegebenenfalls einfach oder mehrfach, gleich oder verschieden substituiertes, geradkettiges oder verzweigtes Alkyl mit 1 bis 6 Kohlenstoffatomen steht, wobei als Alkylsubstituenten infrage kommen:
Halogen, jeweils geradkettiges oder verzweigtes Alkoxy, Alkylthio, Alkylamino oder Dialkylamino mit jeweils 1 bis 4 Kohlenstoffatomen in den einzelnen Alkylteilen;
außerdem für Cycloalkyl mit 3 bis 8 Kohlenstoffatomen steht;

$R^2$ für Wasserstoff, Hydroxy, Mercapto, Amino, Halogen oder für gegebenenfalls einfach oder mehrfach, gleich oder verschieden substituiertes, geradkettiges oder verzweigtes Alkyl mit 1 bis 6 Kohlenstoffatomen steht, wobei als Alkylsubstituenten infrage kommen:
Halogen, Cyano sowie jeweils geradkettiges oder verzweigtes Alkoxy, Alkylthio, Alkylamino oder Dialkylamino mit jeweils 1 bis 4 Kohlenstoffatomen In den einzelnen Alkylteilen,
außerdem für jeweils geradkettiges oder verzweigtes Alkenyl oder Alkinyl mit jeweils 2 bis 6 Kohlenstoffatomen, für jeweils geradkettiges oder verzweigtes Alkoxy, Alkylthio, Alkylsulfinyl oder Alkylsulfonyl mit jeweils 1 bis 6 Kohlenstoffatomen oder für Cycloalkyl mit 3 bis 8 Kohlenstoffatomen steht;
außerdem für gegebenenfalls im Arylteil einfach oder mehrfach, gleich oder verschieden substituiertes Arylalkyl mit 6 bis 10 Kohlenstoffatomen im Arylteil und 1 bis 6 Kohlenstoffatomen im geradkettigen oder verzweigten Alkylteil steht, wobei als Arylsubstituenten infrage kommen:
Halogen, Cyano, Nitro, jeweils geradkettiges oder verzweigtes Alkyl, Alkoxy oder Alkylthio mit jeweils 1 bis 4 Kohlenstoffatomen, jeweils geradkettiges oder Verzweigtes Halogenalkyl, Halogenalkoxy, Halogenalkylthio mit jeweils 1 bis 4 Kohlenstoffatomen und 1 bis 9 gleichen oder verschiedenen Halogenatomen, jeweils geradkettiges oder verzweigtes Alkoxycarbonyl oder Alkoximinoalkyl mit jeweils 1 bis 4 Kohlenstoffatomen in den einzelnen Alkylteilen sowie gegebenenfalls einfach oder mehrfach, gleich oder verschieden durch Halogen und/oder geradkettiges oder verzweigtes Alkyl mit 1 bis 4 Kohlenstoffatomen substituiertes Phenyl;
und außerdem für einen Rest

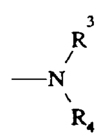

steht,

Het für einen gegebenenfalls einfach oder mehrfach, gleich oder verschieden substituierten Heterocyclus der Formel

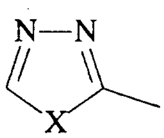 ; 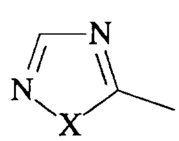 ; 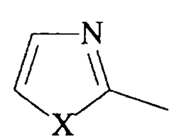 oder 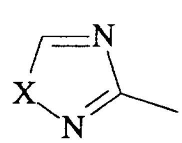

steht,
wobei als Substituenten jeweils infrage kommen:
Halogen, Hydroxy, Mercapto, Amino, Cyano, Nitro, Carbamoyl, jeweils geradkettiges oder verzweigtes Alkyl, Alkoxy, Alkylthio, Alkylsulfinyl, Alkylsulfonyl, Alkylamino, Dialkylamino, Alkanoylamino; Halogenalkylcarbonylamino oder Alkylaminocarbonylamino mit jeweils 1 bis 8 Kohlenstoffatomen in den einzelnen Alkylteilen und gegebenenfalls 1 bis 13 gleichen oder verschiedenen Halogenatomen, jeweils geradkettiges oder verzweigtes Alkenyl oder Alkinyl mit jeweils 2 bis 6 Kohlenstoffatomen, geradkettiges oder verzweigtes Alkylidenamino mit 1 bis 6 Kohlenstoffatomen, jeweils geradkettiges oder verzweigtes Halogenalkyl, Halogenalkoxy, Halogenalkylthio, Halogenalkylsulfinyl oder Halogenalkylsulfonyl mit jeweils 1 bis 6 Kohlenstoffatomen und 1 bis 13 gleichen oder verschiedenen Halogenatomen, geradkettiges oder verzweigtes Alkoxycarbonyl mit 1 bis 7 Kohlenstoffatomen, Cycloalkyl mit 3 bis 8 Kohlenstoffatomen sowie jeweils gegebenenfalls im Arylteil einfach oder mehrfach, gleich oder verschieden durch Halogen und/oder geradkettiges oder verzweigtes Alkyl und/oder Alkoxy mit jeweils 1 bis 4 Kohlenstoffatomen und/oder Halogenalkyl mit 1 bis 4 Kohlenstoffatomen und 1 bis 9 gleichen oder verschiedenen Halogenatomen substituiertes Aryl, Arylalkyl, Arylalkylidenamino oder Arylalkylaminocarbonylamino mit jeweils 6 bis 10 Kohlenstoffatomen im Arylteil und gegebenenfalls 1 bis 4 Kohlenstoffatomen im geradkettigen oder verzweigten Alkylteil und

X jeweils für Sauerstoff, Schwefel, eine NH-Gruppe oder eine geradkettige oder verzweigte N-Alkylgruppe mit 1 bis 8 Kohlenstoffatomen steht,

$R^3$ für Wasserstoff oder für gegebenenfalls einfach oder mehrfach, gleich oder verschieden substituiertes, geradkettiges oder verzweigtes Alkyl mit 1 bis 6 Kohlenstoffatomen steht, wobei als Alkylsubstituenten infrage kommen:
Halogen, Cyano, jeweils geradkettiges oder verzweigtes Alkoxy, Alkylthio, Alkylamino oder Dialkylamino mit jeweils 1 bis 4 Kohlenstoffatomen in den einzelnen Alkylteilen sowie gegebenenfalls einfach oder mehrfach, gleich oder verschieden durch Halogen und/oder geradkettiges oder verzweigtes Alkyl mit 1 bis 4 Kohlenstoffatomen substituiertes Phenyl;
außerdem für jeweils geradkettiges oder verzweigtes Alkenyl oder Alkinyl mit jeweils 2 bis 6 Kohlenstoffatomen, für geradkettiges oder verzweigtes Alkoxy mit 1 bis 6 Kohlenstoffatomen oder für Cycloalkyl mit 3 bis 8 Kohlenstoffatomen steht und

$R^4$ für gegebenenfalls einfach oder mehrfach, gleich oder verschieden substituiertes, geradkettiges oder verzweigtes Alkyl mit 1 bis 6 Kohlenstoffatomen steht, wobei als Alkylsubstituenten infrage kommen:
Halogen, Cyano, jeweils geradkettiges oder verzweigtes Alkoxy, Alkylthio, Alkylamino oder Dialkylamino mit jeweils 1 bis 4 Kohlenstoffatomen in den einzelnen Alkylteilen sowie gegebenenfalls einfach oder mehrfach, gleich oder verschieden durch Halogen und/oder geradkettiges oder verzweigtes Alkyl mit 1 bis 4 Kohlenstoffatomen substituiertes Phenyl;
außerdem für jeweils geradkettiges oder verzweigtes Alkenyl oder Alkinyl mit jeweils 2 bis 6 Kohlenstoffatomen, für geradkettiges oder verzweigtes Alkoxy mit 1 bis 6 Kohlenstoffatomen oder für Cycloalkyl mit 3 bis 8 Kohlenstoffatomen steht oder

$R^3$ und $R^4$ gemeinsam mit dem Stickstoffatom, an welches sie gebunden sind, für einen gegebenenfalls einfach oder mehrfach, gleich oder verschieden substituierten, gesättigten oder ungesättigten Heterocyclus stehen der gegebenenfalls 1 bis 3 weitere Heteroatome - insbesondere Stickstoff, Sauerstoff und/oder Schwefel - enthalten kann, wobei als Heterocyclylsubstituenten jeweils infrage kommen:
Halogen, Hydroxy, Mercapto, Amino, Cyano, Nitro, Carbamoyl, jeweils geradkettiges oder verzweigtes Alkyl, Alkoxy, Alkylthio, Alkylsulfinyl, Alkylsulfonyl, Alkylamino, Dialkylamino, Alkanoylamino; Halogenalkylcarbonylamino oder Alkylaminocarbonylamino mit jeweils 1 bis 8 Kohlenstoffatomen in den einzelnen Alkylteilen und gegebenenfalls 1 bis 13 gleichen oder verschiedenen Halogenatomen, jeweils geradkettiges oder verzweigtes Alkenyl oder Alkinyl mit jeweils 2 bis 6 Kohlenstoffatomen, geradkettiges oder verzweigtes Alkylidenamino mit 1 bis 6 Kohlenstoffatomen, jeweils geradkettiges oder verzweigtes Halogenalkyl, Halogenalkoxy, Halogenalkylthio, Halogenalkylsulfinyl oder Halogenalkylsulfonyl mit jeweils 1 bis 6 Kohlenstoffatomen und 1 bis 13 gleichen

oder verschiedenen Halogenatomen, geradkettiges oder verzweigtes Alkoxycarbonyl mit 1 bis 7 Kohlenstoffatomen, Cycloalkyl mit 3 bis 8 Kohlenztoffatomen sowie jeweils gegebenenfalls im Arylteil einfach oder mehrfach, gleich oder verschieden durch Halogen und/oder geradkettiges oder verzweigtes Alkyl und/oder Alkoxy mit jeweils 1 bis 4 Kohlenstoffatomen und/oder Halogenalkyl mit 1 bis 4 Kohlenstoffatomen und 1 bis 9 gleichen oder verschiedenen Halogenatomen substituiertes Aryl, Arylalkyl, Arylalkylidenamino oder Arylalkylaminocarbonylamino mit jeweils 6 bis 10 Kohlenstoffatomen im Arylteil und gegebenenfalls 1 bis 4 Kohlenstoffatomen im geradkettigen oder verzweigten Alkylteil.

**3.** Heterocyclyltriazolinone der allgemeinen Formel (I) gemäß Anspruch 1, dadurch gekennnzeichnet, daß

$R^1$ für Wasserstoff oder für gegebenenfalls einfach bis zweifach, gleich oder verschieden substituiertes, geradkettiges oder verzweigtes Alkyl mit 1 bis 4 Kohlenstoffatomen steht, wobei als Alkylsubstituenten infrage kommen:
jeweils geradkettiges oder verzweigtes Alkoxy, Alkylthio, Alkylamino oder Dialkylamino mit jeweils 1 bis 3 Kohlenstoffatomen in den einzelnen Alkylteilen;
außerdem für geradkettiges oder verzweigtes Halogenalkyl mit 1 bis 4 Kohlenstoffatomen und 1 bis 9 gleichen oder verschiedenen Halogenatomen - insbesondere Fluor, Chlor und/oder Brom- steht, außerdem für Cycloalkyl mit 3 bis 7 Kohlenstoffatomen steht;

$R^2$ für Wasserstoff, Hydroxy, Mercapto, Amino, Halogen oder für gegebenenfalls einfach oder zweifach, gleich oder verschieden substituiertes, geradkettiges oder verzweigtes Alkyl mit 1 bis 4 Kohlenstoffatomen steht, wobei als Alkylsubstituenten infrage kommen:
Cyano oder jeweils geradkettiges oder verzweigtes Alkoxy, Alkylthio, Alkylamino oder Dialkylamino mit jeweils 1 bis 3 Kohlenstoffatomen in den einzelnen Alkylteilen,
außerdem für geradkettiges oder verzweigtes Halogenalkyl mit 1 bis 4 Kohlenstoffatomen und 1 bis 9 gleichen oder verschiedenen Halogenatomen - insbesondere Fluor, Chlor und/oder Brom- sieht, außerdem für jeweils geradkettiges oder verzweigtes Alkenyl oder Alkinyl mit jeweils 2 bis 5 Kohlenstoffatomen, für jeweils geradkettiges oder verzweigtes Alkoxy, Alkylthio, Alkylsulfinyl oder Alkylsulfonyl mit jeweils 1 bis 4 Kohlenstoffatomen oder für Cycloalkyl mit 3 bis 7 Kohlenstoffatomen steht;
außerdem für gegebenenfalls im Arylteil einfach bis dreifach, gleich oder verschieden substituiertes Arylalkyl mit 6 oder 10 Kohlenstoffatomen im Arylteil und 1 bis 4 Kohlenstoffatomen im geradkettigen oder verzweigten Alkylteil steht, wobei als Arylsubstituenten infrage kommen:
Halogen, Cyano, Nitro, jeweils geradkettiges oder verzweigtes Alkyl, Alkoxy oder Alkylthio mit jeweils 1 bis 3 Kohlenstoffatomen, jeweils geradkettiges oder verzweigtes Halogenalkyl, Halogenalkoxy, Halogenalkylthio mit jeweils 1 bis 3 Kohlenstoffatomen und 1 bis 7 gleichen oder verschiedenen Halogenatomen, jeweils geradkettiges oder verzweigtes Alkoxycarbonyl oder Alkoximinoalkyl mit jeweils 1 bis 3 Kohlenstoffatomen in den einzelnen Alkylteilen sowie gegebenenfalls einfach bis dreifach, gleich oder verschieden durch Halogen und/oder geradkettiges oder verzweigtes Alkyl mit 1 bis 3 Kohlenstoffatomen substituiertes Phenyl;
und außerdem für einen Rest

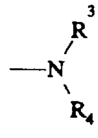

steht,

Het für einen gegebenenfalls einfach bis zweifach, gleich oder verschieden substituierten Heterocyclus der Formel

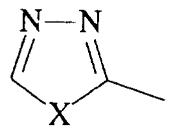 ; 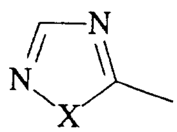 ; 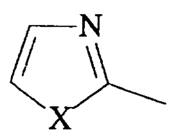 oder 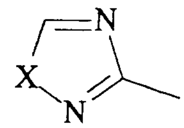

steht, wobei als Substituenten jeweils infrage kommen:
Halogen, Hydroxy, Mercapto, Amino, Cyano, Nitro, Carbamoyl, jeweils geradkettiges oder verzweigtes Alkyl, Alkoxy, Alkylthio, Alkylsulfinyl, Alkylsulfonyl, Alkylamino, Dialkylamino, Alkanoylamino, Halogenalkylcarbonylamino oder Alkylaminocarbonylamino mit jeweils 1 bis 6 Kohlenstoffatomen in den einzelnen Alkylteilen und gegebenenfalls 1 bis 13 gleichen oder verschiedenen Halogenatomen - insbesondere Fluor, Chlor und/oder Brom -, jeweils geradkettiges oder verzweigtes Alkenyl oder Alkinyl mit jeweils 2 bis 5 Kohlenstoffatomen, geradkettiges oder verzweigtes Alkylidenamino mit 1 bis 4 Kohlenstoffatomen, jeweils geradkettiges oder verzweigtes Halogenalkyl, Halogenalkoxy, Halogenalkylthio, Halogenalkylsulfinyl oder Halogenalkylsulfonyl mit jeweils 1 bis 4 Kohlenstoffatomen und 1 bis 9 gleichen oder verschiedenen Halogenatomen, geradkettiges oder verzweigtes Alkoxycarbonyl mit 1 bis 5 Kohlenstoffatomen, Cycloalkyl mit 3 bis 7 Kohlenstoffatomen sowie jeweils gegebenenfalls im Arylteil einfach bis dreifach, gleich oder verschieden durch Halogen und/oder geradkettiges oder verzweigtes Alkyl und/oder Alkoxy mit jeweils 1 bis 3 Kohlenstoffatomen und/oder Halogenalkyl mit 1 bis 3 Kohlenstoffatomen und 1 bis 7 gleichen oder verschiedenen Halogenatomen substituiertes Aryl, Arylalkyl, Arylalkylidenamino oder Arylalkylaminocarbonylamino mit jeweils 6 oder 10 Kohlenstoffatomen im Arylteil und gegebenenfalls 1 bis 3 Kohlenstoffatomen im geradkettigen oder verzweigten Alkylteil und

X jeweils für Sauerstoff, Schwefel, eine NH-Gruppe oder eine geradkettige oder verzweigte N-Alkylgruppe mit 1 bis 6 Kohlenstoffatomen steht,

$R^3$ für Wasserstoff oder für gegebenenfalls einfach bis zweifach, gleich oder verschieden substituiertes, geradkettiges oder verzweigtes Alkyl mit 1 bis 4 Kohlenstoffatomen steht, wobei als Alkylsubstituenten infrage kommen:
Cyano, jeweils geradkettiges oder verzweigtes Alkoxy, Alkylthio, Alkylamino oder Dialkylamino mit jeweils 1 bis 3 Kohlenstoffatomen in den einzelnen Alkylteilen sowie gegebenenfalls einfach bis dreifach, gleich oder verschieden durch Halogen und/oder geradkettiges oder verzweigtes Alkyl mit 1 bis 3 Kohlenstoffatomen substituiertes Phenyl;
außerdem für geradkettiges oder verzweigtes Halogenalkyl mit 1 bis 4 Kohlenstoffatomen und 1 bis 9 gleichen oder verschiedenen Halogenatomen - insbesondere Fluor, Chlor und/oder Brom- steht, außerdem für jeweils geradkettiges oder verzweigtes Alkenyl oder Alkinyl mit jeweils 2 bis 5 Kohlenstoffatomen, für geradkettiges oder verzweigtes Alkoxy mit 1 bis 4 Kohlenstoffatomen oder für Cycloalkyl mit 3 bis 7 Kohlenstoffatomen steht und

$R^4$ für gegebenenfalls einfach oder zweifach, gleich oder verschieden substituiertes, geradkettiges oder verzweigtes Alkyl mit 1 bis 4 Kohlenstoffatomen steht, wobei als Alkylsubstituenten infrage kommen:
Cyano, jeweils geradkettiges oder verzweigtes Alkoxy, Alkylthio, Alkylamino oder Dialkylamino mit jeweils 1 bis 3 Kohlenstoffatomen in den einzelnen Alkylteilen sowie gegebenenfalls einfach bis dreifach, gleich oder verschieden durch Halogen und/oder geradkettiges oder verzweigtes Alkyl mit 1 bis 3 Kohlenstoffatomen substituiertes Phenyl;
außerdem für geradkettiges oder verzweigtes Halogenalkyl mit 1 bis 4 Kohlenstoffatomen und 1 bis 9 gleichen oder verschiedenen Halogenatomen - insbesondere Fluor, Chlor und/oder Brom- steht, außerdem für jeweils geradkettiges oder verzweigtes Alkenyl oder Alkinyl mit jeweils 2 bis 5 Kohlenstoffatomen, für geradkettiges oder verzweigtes Alkoxy mit 1 bis 4 Kohlenstoffatomen oder für Cycloalkyl mit 3 bis 7 Kohlenstoffatomen steht oder

$R^3$ und $R^4$ gemeinsam mit dem Stickstoffatom, an welches sie gebunden sind, für einen gegebenenfalls einfach oder zweifach, gleich oder verschieden substituierten, gesättigten

fünf- bis siebengliedrigen monocyclischen Heterocyclus stehen der gegebenenfalls 1 oder 2 weitere Heteroatome - insbesondere Stickstoff, Sauerstoff und/oder Schwefel - enthalten kann, wobei als Heterocyclylsubstituenten jeweils infrage kommen:
Halogen, Hydroxy, Mercapto, Amino, Cyano, Nitro, Carbamoyl, jeweils geradkettiges oder verzweigtes Alkyl, Alkoxy, Alkylthio, Alkylsulfinyl, Alkylsulfonyl, Alkylamino, Dialkylamino, Alkanoylamino, Halogenalkylcarbonylamino oder Alkylaminocarbonylamino mit jeweils 1 bis 6 Kohlenstoffatomen in den einzelnen Alkylteilen und gegebenenfalls 1 bis 13 gleichen oder verschiedenen Halogenatomen - insbesondere Fluor, Chlor und/oder Brom -, jeweils geradkettiges oder verzweigtes Alkenyl oder Alkinyl mit jeweils 2 bis 5 Kohlenstoffatomen, geradkettiges oder verzweigtes Alkylidenamino mit 1 bis 4 Kohlenstoffatomen, jeweils geradkettiges oder verzweigtes Halogenalkyl, Halogenalkoxy, Halogenalkylthio, Halogenalkylsulfinyl oder Halogenalkylsulfonyl mit jeweils 1 bis 4 Kohlenstoffatomen und 1 bis 9 gleichen oder verschiedenen Halogenatomen, geradkettiges oder verzweigtes Alkoxycarbonyl mit 1 bis 5 Kohlenstoffatomen, Cycloalkyl mit 3 bis 7 Kohlenstoffatomen sowie jeweils gegebenenfalls im Arylteil einfach bis dreifach, gleich oder verschieden durch Halogen und/oder geradkettiges oder verzweigtes Alkyl und/oder Alkoxy mit jeweils 1 bis 3 Kohlenstoffatomen und/oder Halogenalkyl mit 1 bis 3 Kohlenstoffatomen und 1 bis 7 gleichen oder verschiedenen Halogenatomen substituiertes Aryl, Arylalkyl, Arylalkylidenamino oder Arylalkylaminocarbonylamino mit jeweils 6 oder 10 Kohlenstoffatomen im Arylteil und gegebenenfalls 1 bis 3 Kohlenstoffatomen im geradkettigen oder verzweigten Akylteil.

**4.** Heterocyclyltriazolinone der allgemeinen Formel (I) gemäß Anspruch 1, dadurch gekennzeichnet, daß

$R^1$ für Wasserstoff oder für gegebenenfalls einfach substituiertes, geradkettiges oder verzweigtes Alkyl mit 1 bis 3 Kohlenstoffatomen steht, wobei als Alkylsubstituenten infrage kommen:
Alkoxy, Alkylthio, Alkylamino oder Dialkylamino mit jeweils 1 oder 2 Kohlenstoffatomen in den einzelnen Alkylteilen;
außerdem für geradkettiges oder verzweigtes Halogenalkyl mit 1 bis 3 Kohlenstoffatomen und 1 bis 7 gleichen oder verschiedenen Halogenatomen - insbesondere Fluor, Chlor und/oder Brom- steht oder für Cycloalkyl mit 3 bis 6 Kohlenstoffatomen steht;

$R^2$ für Wasserstoff, Hydroxy, Mercapto, Amino, Fluor, Chlor, Brom oder für gegebenenfalls einfach substituiertes, geradkettiges oder verzweigtes Alkyl mit 1 bis 3 Kohlenstoffatomen steht, wobei als Alkylsubstituenten infrage kommen:
Cyano sowie Alkoxy, Alkylthio, Alkylamino oder Dialkylamino mit jeweils 1 oder 2 Kohlenstoffatomen in den einzelnen Alkylteilen;
außerdem für geradkettiges oder verzweigtes Halogenalkyl mit 1 bis 3 Kohlenstoffatomen und 1 bis 7 gleichen oder verschiedenen Halogenatomen - insbesondere Fluor, Chlor und/oder Brom- steht, außerdem für jeweils geradkettiges oder verzweigtes Alkenyl oder Alkinyl mit jeweils 2 bis 4 Kohlenstoffatomen, für jeweils geradkettiges oder verzweigtes Alkoxy, Alkylthio, Alkylsulfinyl oder Alkylsulfonyl mit jeweils 1 bis 3 Kohlenstoffatomen oder für Cycloalkyl mit 3 bis 6 Kohlenstoffatomen steht;
außerdem für gegebenenfalls im Phenylteil einfach oder zweifach, gleich oder verschieden substituiertes Phenylalkyl mit 1 oder 2 Kohlenstoffatomen im Alkylteil steht, wobei als Phenylsubstituenten infrage kommen:
Halogen, Cyano, Nitro, Alkyl, Alkoxy oder Alkylthio mit jeweils 1 oder 2 Kohlenstoffatomen, Halogenalkyl, Halogenalkoxy oder Halogenalkylthio mit jeweils 1 oder 2 Kohlenstoffatomen und 1 bis 5 gleichen oder verschiedenen Halogenatomen - insbesondere Fluor, Chlor und/oder Brom -, Alkoxycarbonyl oder Alkoximinoalkyl mit 1 bis 3 Kohlenstoffatomen sowie gegebenenfalls einfach oder zweifach, gleich oder verschieden durch Fluor, Chlor, Brom, Methyl und/oder Ethyl substituiertes Phenyl;
und außerdem für einen Rest

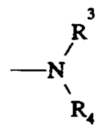

steht,

Het für einen gegebenenfalls einfach oder zweifach, gleich oder verschieden substituierten Heterocyclus der Formel

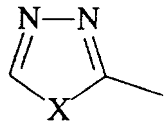 ; 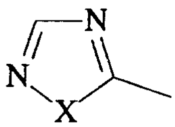 ; 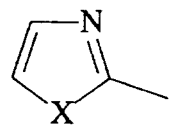 oder 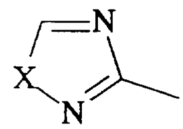

steht,

wobei als Substituenten jeweils infrage kommen:

Halogen, Hydroxy, Mercapto, Amino, Cyano, Nitro, Carbamoyl, jeweils geradkettiges oder verzweigtes Alkyl, Alkoxy, Alkylthio, Alkylsulfinyl, Alkylsulfonyl, Alkylamino, Dialkylamino, Alkanoylamino, Halogenalkylcarbonylamino oder Alkylaminocarbonylamino mit jeweils 1 bis 4 Kohlenstoffatomen in den einzelnen Alkylteilen und gegebenenfalls 1 bis 7 gleichen oder verschiedenen Halogenatomen - insbesondere Fluor, Chlor und/ oder Brom -, jeweils geradkettiges oder verzweigtes Alkenyl oder Alkinyl mit jeweils 2 bis 4 Kohlenstoffatomen, geradkettiges oder verzweigtes Alkylidenamino mit 1 bis 3 Kohlenstoffatomen, jeweils geradkettiges oder verzweigtes Halogenalkyl, Halogenalkoxy, Halogenalkylthio, Halogenalkylsulfinyl oder Halogenalkylsulfonyl mit jeweils 1 bis 3 Kohlenstoffatomen und 1 bis 7 gleichen oder verschiedenen Halogenatomen, geradkettiges oder verzweigtes Alkoxycarbonyl mit 1 bis 4 Kohlenstoffatomen, Cycloalkyl mit 3 bis 6 Kohlenstoffatomen sowie jeweils gegebenenfalls im Phenylteil einfach oder zweifach, gleich oder verschieden durch Fluor, Chlor, Brom, Methyl, Ethyl, Methoxy, Ethoxy und/oder Trifluormethyl substituiertes Phenyl, Benzyl, Phenylethyl, Benzylidenamino oder Benzylaminocarbonylamino und

X für Sauerstoff, Schwefel, eine NH-Gruppe eine N-Methylgruppe oder eine N-Ethylgruppe steht, wobei

$R^3$ für Wasserstoff oder für gegebenenfalls einfach substituiertes, geradkettiges oder verzweigtes Alkyl mit 1 bis 3 Kohlenstoffatomen steht, wobei als Alkylsubstituenten infrage kommen:

Cyano, Alkoxy, Alkylthio, Alkylamino oder Dialkylamino mit jeweils 1 oder 2 Kohlenstoffatomen in den einzelnen Alkylteilen sowie gegebenenfalls einfach oder zweifach, gleich oder verschieden durch Fluor, Chlor, Brom, Methyl und/oder Ethyl substituiertes Phenyl;

außerdem für geradkettiges oder verzweigtes Halogenalkyl mit 1 bis 3 Kohlenstoffatomen und 1 bis 7 gleichen oder verschiedenen Halogenatomen - insbesondere Fluor, Chlor und/oder Brom- steht, außerdem für jeweils geradkettiges oder verzweigtes Alkenyl oder Alkinyl mit jeweils 2 bis 4 Kohlenstoffatomen, für geradkettiges oder verzweigtes Alkoxy mit 1 bis 3 Kohlenstoffatomen oder für Cycloalkyl mit 3 bis 6 Kohlenstoffatomen steht und

$R^4$ für gegebenenfalls einfach substituiertes, geradkettiges oder verzweigtes Alkyl mit 1 bis 3 Kohlenstoffatomen steht, wobei als Alkylsubstituenten infrage kommen:

Cyano, Alkoxy, Alkylthio, Alkylamino oder Dialkylamino mit jeweils 1 oder 2 Kohlenstoffatomen in den einzelnen Alkylteilen sowie gegebenenfalls einfach oder zweifach, gleich oder verschieden durch Fluor, Chlor, Brom, Methyl und/oder Ethyl substituiertes Phenyl;

außerdem für geradkettiges oder verzweigtes Halogenalkyl mit 1 bis 3 Kohlenstoffatomen und 1 bis 7 gleichen oder verschiedenen Halogenatomen - insbesondere Fluor, Chlor und/oder Brom- steht, außerdem für jeweils geradkettiges oder verzweigtes Alkenyl oder Alkinyl mit jeweils 2 bis 4 Kohlenstoffatomen, für geradkettiges oder

verzweigtes Alkoxy mit 1 bis 3 Kohlenstoffatomen oder für Cycloalkyl mit 3 bis 6 Kohlenstoffatomen steht oder

$R^3$ und $R^4$ gemeinsam mit dem Stickstoffatom, an welches sie gebunden sind, für einen gegebenenfalls einfach oder zweifach, gleich oder verschieden durch Methyl und/oder Ethyl und/oder Methoxy substituierten 1-Pyrrolidinyl-, 1-Piperidinyl, 1-Perhydroazepinyl oder 4-Morpholinylrest stehen.

**5.** Verfahren zur Herstellung von Heterocyclyltriazolinonen der allgemeinen Formel (I)

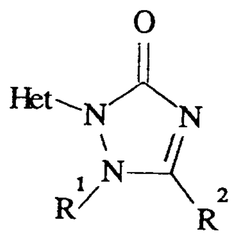

(I)

in welcher

$R^1$ für Wasserstoff oder für jeweils gegebenenfalls substituiertes Alkyl oder Cycloalkyl steht,

$R^2$ für Wasserstoff, Hydroxy, Mercapto, Halogen oder für jeweils gegebenenfalls substituiertes Alkyl, Alkenyl, Alkinyl, Cycloalkyl, Alkoxy, Alkylthio, Alkylsulfinyl, Alkylsulfonyl, Amino oder Heterocyclyl steht und

Het für einen gegebenenfalls substituierten Heterocyclus der Formel

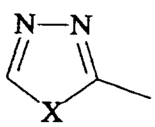 ; 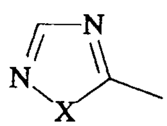 ; 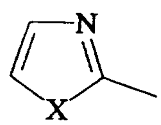 oder 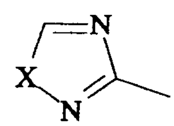

steht, wobei

X jeweils für Sauerstoff, Schwefel, eine NH-Gruppe oder eine N-Alkylgruppe steht,

dadurch gekennzeichnet, daß man

Heterocyclen der Formel (II),

Het-E (II)

in welcher

Het die oben angegebene Bedeutung hat und

E für eine elektronenanziehende Abgangsgruppe steht,

mit Triazolinonen der Formel (III),

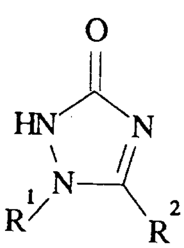

(III)

in welcher

$R^1$ und $R^2$ die oben angegebene Bedeutung haben,

gegebenenfalls in Gegenwart eines Verdünnungsmittels und gegebenenfalls in Gegenwart eines Reaktionshilfsmittels umsetzt.

6. Herbizide Mittel, gekennzeichnet durch einen Gehalt an mindestens einem Heterocyclyltriazolinon der allgemeinen Formel (I) gemäß der Ansprüche 1 bis 5.

7. Verfahren zur Bekämpfung von unerwünschten Pflanzen, dadurch gekennzeichnet, daß man Heterocyclyltriazolinone der allgemeinen Formel (I) gemäß der Ansprüche 1 bis 5 auf Pflanzen und/oder ihren Lebensraum einwirken läßt.

8. Verwendung von Heterocyclyltriazolinonen der allgemeinen Formel (I) gemäß der Ansprüche 1 bis 5 zur Bekämpfung von unerwünschten Pflanzen.

9. Verfahren zur Herstellung von herbiziden Mitteln, dadurch gekennzeichnet, daß man Heterocyclyltriazolinone der allgemeinen Formel (I) gemäß der Ansprüche 1 bis 5 mit Streckmitteln und/oder oberflächenaktiven Substanzen vermischt.